# EUROPEAN PATENT SPECIFICATION

(11) **EP 4 023 278 B1**
(45) Date of publication and mention of the grant of the patent: **10.12.2025**
(21) Application number: 20856207.4
(22) Date of filing: 05.08.2020
(51) Int. Cl.: A61M 25/00, A61N 5/06

(54) **CATHETER AND LIGHT IRRADIATION SYSTEM**
KATHETER UND LICHTBESTRAHLUNGSSYSTEM
CATHÉTER ET SYSTÈME D'EXPOSITION À LA LUMIÈRE

(30) Priority: 30.08.2019 JP 2019158317
(43) Date of publication of application: 06.07.2022
(73) Proprietor: ASAHI INTECC CO., LTD., Seto-shi, Aichi 489-0071 (JP)
(72) Inventor: TSUKAMOTO Toshihiko, Seto-shi, Aichi 489-0071 (JP); KATSURADA Yuko, Seto-shi, Aichi 489-0071 (JP); UTANI Takayuki, Seto-shi, Aichi 489-0071 (JP); MATSUSHIMA Kazuo, Seto-shi, Aichi 489-0071 (JP)
(74) Representative: Prinz & Partner mbB
(86) International application number: PCT/JP2020/029973
(87) International publication number: WO 2021/039323

(56) References cited:
- JP-A- 2001 129 094
- JP-A- 2005 534 409
- JP-A- 2006 271 831
- JP-A- 2012 504 019
- JP-A- 2015 073 705
- JP-A- H05 103 753
- US-A1- 2005 128 742
- US-A1- 2005 165 279
- US-A1- 2006 064 009
- US-A1- 2015 174 379

## Description

### TECHNICAL FIELD

The present invention relates to a catheter and a light irradiation system.

### BACKGROUND ART

In cancer treatment, surgical, radiological, and drug (chemical) methods are used alone or in combination, and each technique has been developed in recent years. However, there are many cancers for which a satisfactory treatment technique has not yet been found, and further development of the treatment technique is expected. As one of the cancer treatment techniques, a method referred to as PDT (Photodynamic Therapy) is known. PDT involves intravenous administration of a photosensitizer followed by light irradiation to generate active oxygen in cancer cells, thereby killing the cancer cells (see, for example, Makoto Mitsunaga, Mikako Ogawa, Nobuyuki Kosaka Lauren T. Rosenblum, Peter L. Choyke, and Hisataka Kobayashi: "Cancer Cell-Selective In Vivo Near Infrared Photoimmunotherapy Targeting Specific Membrane Molecules", Nature Medicine, 2012, 17(12), p. 1685-1691). However, PDT is problematic in that a photosynthesizer has low selectivity for accumulation in cancer cells, so as to be incorporated into normal cells, resulting in significant side effects. Hence, PDT is not widely used as a treatment technique.

NIR-PIT (Near-infrared photoimmunotherapy) is a treatment technique that has been attracting attention in recent years. NIR-PIT involves the use of a complex which is prepared by linking two compounds, an antibody against a specific antigen of cancer cells and a photosensitizer (for example, IRDye700DX). When administered intravenously, this complex selectively accumulates in cancer cells *in vivo.* Then, irradiation with light having an excitation wavelength (for example, 690 nm) of the photosensitizer in the complex activates the complex to exhibit an anticancer effect (see, for example, JP 2014 523907 A). NIR-PIT can reduce side effects as compared with PDT because of the accumulation selectivity for cancer of an antibody and local light irradiation. NIR-PIT further involves light irradiation (NIR irradiation) in a near-infrared region of, for example, 690 nm, and thus the effect of NIR irradiation on the immune system can be expected (see, for example, Kazuhide Sato, Noriko Sato, Biying Xu, Yuko Nakamura, Tadanobu Nagaya, Peter L. Choyke, Yoshinori Hasegawa, and Hisataka Kobayashi: "Spatially selective depletion of tumor-associated regulatory T cells with near-infrared photoimmunotherapy", Science Translational Medicine, 2016, Vol.8, Issue 352, ra110).

The predetermined wavelength region including 690 nm exemplified above is also referred to as a biological spectroscopic window, in which light is absorbed less by biological components than other wavelength regions, but is problematic in that it cannot be applied to cancers deep inside the body due to insufficient light permeability when light irradiation is performed from the body surface. Therefore, in recent years, research has been conducted on NIR-PIT that involves irradiating with light at a position closer to cancer cells instead of irradiating with light from the body surface (see, for example, Shuhei Okuyama, Tadanobu Nagaya, Kazuhide Sato, Fusa Ogata, Yasuhiro Maruoka, Peter L. Choyke, and Hisataka Kobayashi: "Interstitial near-infrared photoimmunotherapy: effective treatment areas and light doses needed for use with fiber optic diffusers", Oncotarget, 2018 Feb 16, 9(13), p.11159-11169). For example, JP 2018 000 867 A and JP 2007 528 752 A disclose devices that can be used in such PDT and NIR-PIT. All of the devices described in JP 2018 000 867 A, JP 2007 528 752 A, JP 2015 073 705 A and JP 2005 534 409 A are inserted into blood vessels and used, and can irradiate a portion deep inside the body with light.

US 2005/128742 A1 discloses an apparatus for illumination a body portion of a body lumen comprising an elongate flexible body, an array of light sources disposed adjacent to a proximal end of the flexible body and means that are not inflatable for reducing an amount of bodily fluid adjacent to the array of light sources.US 2006/0064009 A1 describes an apparatus comprising a cannula for insertion into a vessel of a subject with a lumen opening to the outer surface of the cannula, and a fluid dispersion device coupled to the cannula such to disperse fluid introduced from the lumen opening into a flow of fluid within the vessel.

From US 2005/0165279 A1, an apparatus for providing an image of a target region located within an intravascular environment is known. The apparatus comprises a camera with at least one light sensing region, at least one light source for emitting polarized light on the target region and at least one polarization filter for filtering said light.

US 2015/0174379 A1 shows a catheter for treating a body lumen comprising an elongate member comprising a proximal end, a distal end sized for introduction into a body lumen, a hub on the proximal end, and a fluid delivery lumen communicating between an inlet port of on the hub and a transit port on a distal portion of the elongate member. The catheter further comprises an inflatable infusion element.

### SUMMARY OF THE INVENTION

### TECHNICAL PROBLEM

In PDT and NIR-PIT, as described above, cancer cells in which a complex is accumulated are irradiated with light having the excitation wavelength of the photosensitizer in the complex to kill the cancer cells. Here, a biological lumen is normally filled with a body fluid, such as blood flowing through blood vessels. Therefore, the devices described in JP 2007 528 752 A, JP 2015 073 705 A and JP 2005 534 409 A include lumens for releasing a fluid such as physiological saline in order to eliminate the body fluid in the biological lumen and improve the efficiency of irradiating a living tissue with light. However, the techniques described in JP 2007 528 752 A, JP 2015 073 705 A and JP 2005 534 409 A do not consider the direction in which the fluid is released, which may result in insufficient effects. Further, the techniques described in JP 2014 523907 A and JP 2018 000 867 A do not consider the release of a fluid into a biological lumen.

Such problems are common not only to PDT and NIR-PIT, but also to all devices to be used in examinations or treatments including the process of light irradiation in vivo. Moreover, such a problem is also common not only to devices to be inserted into blood vessels, but also to all devices to be inserted into biological lumens, such as the vascular system, the lymph gland system, the biliary system, the urinary tract system, the airway system, the digestive organ system, secretory glands and reproductive organs.

The present invention has been made to solve at least a part of the above-mentioned problems, and an object thereof is to make it possible to supply a fluid to a light irradiation site in a catheter and a light irradiation system for irradiating the inside of a biological lumen with light.

### SOLUTION TO THE PROBLEM

The present invention has been made to solve at least a part of the problems described above, and can be realized as the following aspects.
(1) According to one aspect of the present invention, a catheter according to claim 1 is provided.

According to this configuration, the catheter includes a light emission part that emits light in a predetermined outward direction, and an opening that is capable of releasing a fluid, wherein the releasing direction of the fluid from the opening intersects the light emission direction from the light emission part. Therefore, the fluid can be directly supplied to the light irradiation site. As a result, for example, when physiological saline is used as the fluid, a body fluid at the light irradiation site can be effectively removed and the concentration of the body fluid can be locally lowered. Specifically, when a catheter is inserted into a blood vessel, a local decrease in blood concentration in the vicinity of the light irradiation site can suppress light absorption by blood, and as a result, this can contribute to the suppression of the occurrence of an event that light does not reach a target tissue. In addition, blood coagulation due to blood overheating can be suppressed. Suppression of blood coagulation can contribute not only to the improvement of safety, but also to the suppression of the occurrence of an event that light is blocked by coagulated (or carbonized) blood, so as to be unable to reach a target tissue. Further, for example, when physiological saline is used as a fluid, the target tissue can be cooled, and the temperature rise of the target tissue due to irradiation with light can be suppressed. Further, for example, when a photosensitizer is used as a fluid, the efficiency of irradiating a target tissue with light can be improved.

(2) In the catheter of the above aspect, the opening includes a first opening arranged on the proximal end side beyond the light emission part, and the fluid may be released from the first opening in such a manner that the direction in which the fluid is released is inclined to the side of the light emission part.

According to this configuration, the first opening as an opening capable of releasing a fluid is arranged on the proximal end side beyond the light emission part, and the fluid is released in such a manner that the direction in which the fluid is released is inclined to the light emission part side. Therefore, through the use of the first opening, the fluid can be released from the proximal end side beyond the light emission part to the light emission part. Further, since the positions of the light emission part and the first opening are different in the long axis direction, the degree of freedom in arranging the light emission part and the inner shaft communicating with the first opening in the catheter can be improved.

(3) In the catheter of any one of the above aspects, the opening includes a second opening arranged on the distal end side beyond the light emission part, and the fluid may be released from the second opening in such a manner that the direction in which the fluid is released is inclined to the side of the light emission part.

According to this configuration, the second opening as an opening capable of releasing a fluid is arranged on the distal end side beyond the light emission part, and the fluid is released in such a manner that the direction in which the fluid is released is inclined to the side of the light emission part. Therefore, through the use of the second opening, the fluid can be released from the distal end side beyond the light emission part to the light emission part. Further, since the positions of the light emission part and the second opening are different in the long axis direction, the degree of freedom in arranging the light emission part and the inner shaft communicating with the second opening in the catheter can be improved.

(4) In the catheter according to the invention, the opening includes a third opening arranged at a position different in the circumferential direction from a position where light is emitted from the light emission part on the side of the main body part, and the fluid may be released from the third opening in such a manner that the direction in which the fluid is released is inclined to the side of the light emission part.

According to this configuration, the third opening as an opening capable of releasing a fluid is arranged at a position different in the circumferential direction from the position where the light is emitted by the light emission part, and the fluid is released in such a manner that the direction in which the fluid is released is inclined to the side of the light emission part. Therefore, through the use of the third opening, the fluid can be released from the position different in the circumferential direction to the light emission part. Further, since the positions of the light emission part and the third opening are different in the circumferential direction, the degree of freedom in arranging the light emission part and the inner shaft communicating with the third opening in the catheter can be improved.

(5) The catheter of any one of the above aspects may further include a first expansion/contraction part that is provided in the main body part, arranged on the proximal end side beyond the first opening, and is expandable and contractable in the radial direction.

According to this configuration, the first expansion/contraction part that is expandable and contractable in the radial direction is further included. Therefore, expanding the first expansion/contraction part can inhibit the flow of a body fluid in a biological lumen, and can efficiently achieve a decrease in the local concentration of the body fluid due to the release of the fluid. Further, expanding the first expansion/contraction part can position (fix) the distal end part of the catheter in the biological lumen.

(6) The catheter of any one of the above aspects may include a second expansion/contraction part that is provided in the main body part, arranged on the distal end side beyond the light emission part, and is expandable and contractable in the radial direction.

According to this configuration, the second expansion/contraction part that is expandable and contractable in the radial direction may be further included. Therefore, expanding the second expansion/contraction part can inhibit the flow of a body fluid in a biological lumen, and can efficiently achieve a decrease in the local concentration of the body fluid due to the release of the fluid. Further, expanding the second expansion/contraction part can position (fix) the distal end part of the catheter in the biological lumen. Further, when a configuration in which both the first expansion/contraction part and the second expansion/contraction part are included is employed, a first opening and a light emission part are provided between the first expansion/contraction part and the second expansion/contraction part. Therefore, expanding the first expansion/contraction part and the second expansion/contraction part can lower the concentration of the body fluid in the vicinity of the light emission part (between the first expansion/contraction part and the second expansion/contraction part) more efficiently, and thus can further improve the efficiency of irradiating a target tissue with light.

(7) The catheter of any one of the above aspects may further include a third expansion/contraction part that is provided in the main body part, arranged on the distal end side beyond the second opening, and is expandable/contractable in the radial direction.

According to this configuration, a third expansion/contraction part that is expandable and contractable in the radial direction is further included. Therefore, expanding the third expansion/contraction part can inhibit the flow of a body fluid in a biological lumen, and can efficiently achieve a decrease in the local concentration of the body fluid due to the release of the fluid. Further, expanding the third expansion/contraction part can position (fix) the distal end part of the catheter in the biological lumen.

(8) The catheter of any one of the above aspects may further include a fourth expansion/contraction part that is provided in the main body part, arranged on the proximal end side beyond the light emission part, and is expandable/contractable in the radial direction.

According to this configuration, the fourth expansion/contraction part that is expandable and contractable in the radial direction is further included. Therefore, expanding the fourth expansion/contraction part can inhibit the flow of a body fluid in a biological lumen, and can efficiently achieve a decrease in the local concentration of the body fluid due to the release of the fluid. Further, expanding the fourth expansion/contraction part can position (fix) the distal end part of the catheter in the biological lumen. Further, when a configuration in which both the third expansion/contraction part and the fourth expansion/contraction part are included is employed, a second opening and a light emission part are provided between the third expansion/contraction part and the fourth expansion/contraction part. Therefore, expanding the third expansion/contraction part and the fourth expansion/contraction part can lower the concentration of the body fluid in the vicinity of the light emission part (between the third expansion/contraction part and the fourth expansion/contraction part) more efficiently, and thus can further improve the efficiency of irradiating a target tissue with light.

(9) The catheter of any one of the above aspects may further include a fifth expansion/contraction part that is provided in the main body part, arranged on the proximal end side beyond the third opening, and is expandable/contractable in the radial direction.

According to this configuration, the fifth expansion/contraction part that is expandable and contractable in the radial direction is further included. Therefore, expanding the fifth expansion/contraction part can inhibit the flow of a body fluid in a biological lumen, and can efficiently achieve a decrease in the local concentration of the body fluid due to the release of the fluid. Further, expanding the fifth expansion/contraction part can position (fix) the distal end part of the catheter in the biological lumen.

(10) The catheter of any one of the above aspects may further include a sixth expansion/contraction part that is provided in the main body part, arranged on the distal end side beyond the light emission part, and is expandable/contractable in the radial direction.

According to this configuration, the sixth expansion/contraction part that is expandable and contractable in the radial direction is further included. Therefore, expanding the sixth expansion/contraction part can inhibit the flow of a body fluid in a biological lumen, and can efficiently achieve a decrease in the local concentration of the body fluid due to the release of the fluid. Further, expanding the sixth expansion/contraction part can position (fix) the distal end part of the catheter in the biological lumen. Further, when a configuration in which both the fifth expansion/contraction part and the sixth expansion/contraction part are included is employed, a third opening and a light emission part are provided between the fifth expansion/contraction part and the sixth expansion/contraction part. Therefore, expanding the fifth expansion/contraction part and the sixth expansion/contraction part can lower the concentration of the body fluid in the vicinity of the light emission part (between the fifth expansion/contraction part and the sixth expansion/contraction part) more efficiently, and thus can further improve the efficiency of irradiating a target tissue with light.

(11) According to one aspect of the present invention, a light irradiation device is provided. The light irradiation device includes the catheter of any one of the above aspects and a light irradiation device to be inserted into the catheter. The light irradiation device includes a light irradiation part for light irradiation, which is provided at a position in proximity to the light emission part when the light irradiation device is inserted into the catheter. The light emission part of the catheter emits light to the outside by allowing the light irradiated by the light irradiation part to pass therethrough, wherein the direction in which the fluid is released from the opening of the catheter intersects the direction in which the light is emitted from the light emission part.

According to this configuration, the light irradiation system includes a catheter and a light irradiation device to be inserted into the catheter, whereby when the light irradiation device is inserted into the catheter, the light emission part of the catheter emits light to the outside by allowing the light irradiated by the light irradiation part of the light irradiation device to pass therethrough. In this way, the catheter and the light irradiation device are separately provided, so that the range of procedures the operator can adopt can be expanded. Further, the releasing direction of the fluid from the opening intersects the emission direction of the light from the light emission part of the catheter, so that the fluid can be directly supplied to the light irradiation site.

The present invention can be realized in various aspects, for example, a catheter, a light irradiation device, a light irradiation system in which these are separately provided or integrated, and methods for producing a catheter, a light irradiation device, and a light irradiation system.

### BRIEF DESCRIPTION OF DRAWINGS

Fig. 1 is an explanatory view illustrating the configuration of the light irradiation system according to a first embodiment.
Fig. 2 is an explanatory view illustrating the use state of a light irradiation system.
Fig. 3 is an explanatory view illustrating a configuration of a light irradiation system according to a second embodiment.
Fig. 4 is an explanatory view illustrating the use state of the light irradiation system according to the second embodiment.
Fig. 5 is an explanatory view illustrating the configuration of a light irradiation system according to a third embodiment.
Fig. 6 is an explanatory view illustrating the use state of the light irradiation system according to the third embodiment.
Fig. 7 is a schematic diagram of a light irradiation system seen from direction A in Fig. 6.
Fig. 8 is an explanatory view illustrating the configuration of the distal end side of a catheter according to a fourth embodiment.
Fig. 9 is an explanatory view illustrating the configuration of the distal end side of a catheter according to a fifth embodiment.
Fig. 10 is an explanatory view illustrating the configuration of the distal end side of a catheter according to a sixth embodiment.
Fig. 11 is an explanatory view illustrating the configuration of the distal end side of a catheter according to a seventh embodiment.
Fig. 12 is an explanatory view illustrating the configuration of the distal end side of a catheter according to the eighth embodiment.
Fig. 13 is an explanatory view illustrating the configuration of the distal end side of a catheter according to a ninth embodiment.
Fig. 14 is an explanatory view illustrating the configuration of the distal end side of a catheter according to a tenth embodiment.
Fig. 15 is an explanatory view illustrating the configuration of a light irradiation system according to an eleventh embodiment.
Fig. 16 is an explanatory view illustrating the configuration of a light irradiation system according to a twelfth embodiment.

### EMBODIMENTS OF THE INVENTION

### <First Embodiment> (not according to the claims)

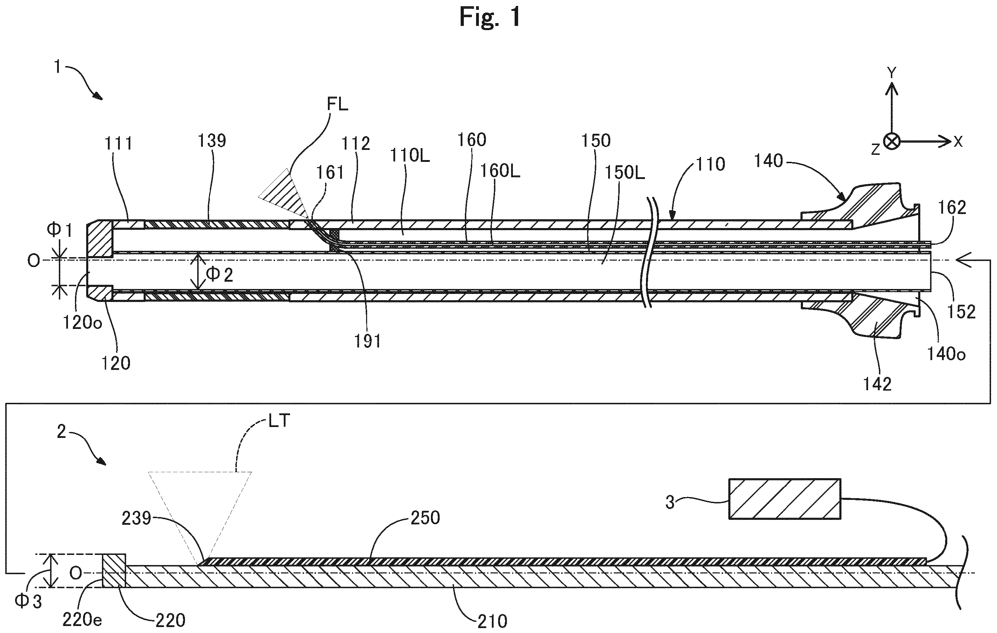

Fig. 1 is an explanatory view illustrating the configuration of the light irradiation system according to the first embodiment. The light irradiation system is a device that is used by inserting it into a biological lumen such as the vascular system, the lymph gland system, the biliary tract system, the urethral system, the airway system, the digestive organ system, secretory glands and reproductive organs, and irradiates a biological tissue with light from the inside of a biological lumen. In the following embodiments, light irradiation systems that are inserted into blood vessels and used to irradiate biological tissues with light from the inside of the blood vessels will be illustrated and described. The light irradiation system can be used in, for example, PDT (Photodynamic Therapy) and NIR-PIT (Near-infrared photoimmunotherapy). In the following embodiments, laser light is illustrated as an example of light, but the light irradiation system may be configured using not only laser light, but also, for example, LED light or white light. The light irradiation system includes a catheter 1 and a light irradiation device 2 that is inserted into and used in the catheter 1. Fig. 1 illustrates the catheter 1 and the light irradiation device 2 separately.

In Fig. 1, the axis passing through the center of the catheter 1 and the axis passing through the center of the light irradiation device 2 are represented by axis lines O (dashed line), respectively. Hereinafter, in a state where the light irradiation device 2 is inserted into the catheter 1, the axes passing through the centers of the two will be described as being different, but the axes passing through the centers of the two may be the same. Further, Fig. 1 illustrates XYZ axes that are orthogonal to each other. The X-axis corresponds to the long axis direction of the catheter 1 and the light irradiation device 2, the Y axis corresponds to the height direction of the catheter 1 and the light irradiation device 2, and the Z axis corresponds to the width direction of the catheter 1 and the light irradiation device 2. The left side (-X-axis direction) of Fig. 1 is referred to as the "distal end side" of the catheter 1, that of the light irradiation device 2, and that of each constituent member, and the right side (+ X-axis direction) of Fig. 1 is referred to as the "proximal end side" of the catheter 1, that of the light irradiation device 2, and that of each constituent member. Further, regarding the catheter 1, the light irradiation device 2, and each constituent member, the end part located on the distal end side is referred to as a "distal end", and the distal end and its vicinity are referred to as a "distal end part". Further, the end part located on the proximal end side is referred to as a "proximal end", and the proximal end and its vicinity are referred to as a "proximal end part". The distal end side is inserted into the living body, and the proximal end side is operated by an operator such as a doctor. These points are also common to the figures illustrating the overall configurations in Fig. 1 and the following figures.

When the catheter 1 is used, the light irradiation device 2 is inserted inside the catheter 1 and light irradiated by the light irradiation device 2 passes through the catheter 1. The catheter 1 has a long tubular shape, and includes a main body part 110, a distal tip 120, a connector 140, an inner shaft 150, and a first inner shaft 160.

The main body part 110 is a long tubular member extending along the axis O. The main body part 110 includes a light emission part 139, a first shaft 111 arranged on the distal end side of the light emission part 139, and a second shaft 112 arranged on the proximal end side of the light emission part 139.

The light emission part 139 allows the laser light inside the main body part 110 (hereinafter, also referred to as "light" or "emitted light") to pass therethrough to the outside, so as to emit light in a predetermined outward direction. The light emission part 139 is a hollow, substantially cylindrical member having a substantially constant outer diameter and inner diameter. In other words, the light emission part 139 is provided in the entire circumferential direction, and allows the light inside the main body part 110 to pass therethrough to the outside in the entire circumferential direction. The light emission part 139 can be formed of an optically transparent resin material, such as an acrylic resin, polyethylene terephthalate, polyvinyl chloride, or the like.

The first shaft 111 is a hollow, substantially cylindrical member having a substantially constant outer diameter and inner diameter. The distal end part of the first shaft 111 is fixed by the distal tip 120 and the proximal end part of the first shaft 111 is joined to the distal end part of the light emission part 139. Joining can be realized by any method, and for example, joining with an adhesive such as an epoxy adhesive or joining with a metal solder such as silver brazing, gold brazing, zinc, Sn-Ag alloy, and Au-Sn alloy can be adopted. Note that the first shaft 111 may be omitted.

The second shaft 112 is a hollow, substantially cylindrical member having a substantially constant outer diameter and inner diameter. The distal end part of the second shaft 112 is joined to the proximal end part of the light emission part 139 and the proximal end part of the second shaft 112 is fixed by a connector 140. The first shaft 111, the second shaft 112, and the light emission part 139 have substantially the same inner diameter and outer diameter, and are joined in a state where the respective inner cavities communicate with each other, thereby configuring the inner cavity 110L communicating from the distal end of the first shaft 111 to the proximal end of the second shaft 112. The outer diameter, inner diameter and length of the first and second shafts 111 and 112 can be arbitrarily determined. A first opening 161 communicating with the inside and outside of the cylinder is provided on the side of the second shaft 112. The first opening 161 leads to a first fluid lumen 160L of the first inner shaft 160, and thus is capable of releasing a fluid supplied through the first fluid lumen 160L to the outside.

The distal tip 120 is a member that is joined to the distal end part of the first shaft 111 and the distal end part of the inner shaft 150 and advances in the blood vessel ahead of other members. The distal end part of the distal tip 120 has an outer shape whose diameter is reduced from the proximal end side to the distal end side in order to facilitate the progress of the catheter 1 in the blood vessel. Further, in the substantially central portion of the distal tip 120, an inner cavity penetrating the distal tip 120 in the axis O direction is formed. In the illustrated example, the diameter Φ1 of the inner cavity of the distal tip 120 is smaller than the diameter Φ2 of the inner cavity (device lumen 150L) of the inner shaft 150. Note that the diameter Φ1 of the inner cavity of the distal tip 120 may be substantially the same as the diameter Φ2 of the device lumen 150L. The opening 120o on the distal end side of the distal tip 120 leads to the inner cavity of the distal tip 120, and is used when a guide wire or the like is inserted into the device lumen 150L. The outer diameter and length of the distal tip 120 can be arbitrarily determined.

The inner shaft 150 is a long tubular member arranged along the axis O. The inner shaft 150 has a hollow substantially cylindrical shape having openings formed at the distal end part and the proximal end part, respectively, and having an inner cavity inside (device lumen 150L) for bringing the two openings into communication. The inner shaft 150 has a smaller diameter than the first and second shafts 111 and 112, and is inserted into the inner cavity 110L of the first and second shafts 111 and 112. The distal end part of the inner shaft 150 is integrally fixed to the distal end part of the first shaft 111 by the distal tip 120. The proximal end part of the inner shaft 150 projects from the proximal end part of the connector 140, and is configured so that another medical device (for example, a guide wire, a light irradiation device 2, etc.) can be taken in and out of the catheter 1. The proximal end part of the inner shaft 150 may be configured so that it is assembled inside the connector 140 and thus the medical device can be taken in and out of the device lumen 150L via the connector 140.

The first inner shaft 160 is a long tubular member arranged along the axis O. The inner shaft 150 has a hollow substantially cylindrical shape having openings formed at the distal end part and the proximal end part, respectively, and having an inner cavity inside (first fluid lumen 160L) for bringing the two openings into communication. The inner shaft 160 has a smaller diameter than the second shaft 112 and the inner shaft 150, and is inserted into the inner cavity 110L of the second shaft 112. The distal end part of the first inner shaft 160 is curved and joined to the second shaft 112 in a state where the first fluid lumen 160L and the first opening 161 communicate with each other. Joining can be realized by any method such as joining with an adhesive or joining with a metal solder. The proximal end part of the first inner shaft 160 projects from the proximal end part of the connector 140, and is configured so that the fluid can be supplied from the opening (opening 162 on the proximal end side) of the proximal end part to the first fluid lumen 160L. The proximal end part of the first inner shaft 160 may be configured so that it is assembled inside the connector 140 and the fluid can be supplied to the first fluid lumen 160L via the connector 140.

The connector 140 is a member that is joined to the proximal end part of the second shaft 112 and is gripped by an operator. The connector 140 has a hollow shape having openings formed at the distal end part and the proximal end part, respectively, and having an inner cavity inside for bringing the two openings into communication. The connector 140 has two blades 142 to be used by the operator to grip the catheter 1. The blades 142 can have any shape. The blades 142 may be omitted, or may be configured to be removable from the connector 140. An opening 140o on the proximal end side of the connector 140 leads to the inner cavity of the connector 140. The outer diameter, inner diameter and length of the connector 140 can be arbitrarily determined.

The first sealing part 191 is a joint member provided at the distal end part of the first inner shaft 160 between the outer peripheral surface of the first inner shaft 160 and the inner peripheral surface of the second shaft 112. The first sealing part 191 is provided to maintain the curved shape of the distal end part of the first inner shaft 160.

The light irradiation device 2 is a probe body for light irradiation, which is inserted into the catheter 1 when it is used. The light irradiation device 2 includes a second main body part 210, a distal tip 220, a light transmission part 250, and a light irradiation part 239.

The second main body part 210 is a long tubular member arranged along the axis O. The second main body part 210 may be hollow or solid. The distal tip 220 is a substantially cylindrical member that is joined to the distal end part of the second main body part 210 and advances ahead of other members in the catheter 1. Here, the outer diameter Φ3 of the distal tip 220 is preferably larger than the diameter Φ1 of the distal tip 120 of the catheter 1 and smaller than the diameter Φ2 of the device lumen 150L of the catheter 1 (Φ1 <Φ3 <Φ2).

The light transmission part 250 is an optical fiber extending along the axis O direction from the distal end side to the proximal end part of the second main body part 210. The light transmission part 250 is joined to the outer surface of the second main body part 210. Joining can be realized by any method, for example, joining with an adhesive such as an epoxy adhesive can be adopted. The proximal end part of the light transmission part 250 is directly or indirectly connected via another optical fiber to a laser light generator 3 that generates laser light with an arbitrary wavelength via a connector. The laser light generator 3 functions as a "light source" installed outside the catheter 1.

The light irradiation part 239 is provided at the distal end part of the light transmission part 250, and performs irradiation with laser light LT (light LT, emitted light LT) transmitted by the light transmission part 250 toward a predetermined outward direction (Fig. 1: light LT denoted with a broken line). The light irradiation part 239 according to the present embodiment is formed by subjecting a core exposed at the distal end part of the light transmission part 250 to a known process, for example, a process of cutting the distal end surface diagonally, a process of forming a notch, a sandblast process, or a chemical process. Note that the light irradiation part 239 may be a resin body that covers the core exposed in the light transmission part 250. In this case, the light irradiation part 239 can be formed by, for example, applying an acrylic ultraviolet curable resin in which quartz fine powder has been dispersed and then curing the resultant with ultraviolet light. Further, the light irradiation part 239 may be a light reflection mirror that reflects light from the core.

The first shaft 111 and the second shaft 112 of the catheter 1, the inner shaft 150 and the first inner shaft 160 of the catheter 1, and the second main body part 210 of the light irradiation device 2 are preferably antithrombotic, flexible, and biocompatible, and can be formed of a resin material or a metal material. As the resin material, a polyamide resin, a polyolefin resin, a polyester resin, a polyurethane resin, a silicone resin, a fluororesin, and the like can be adopted, for example. Examples of the metal material that can be adopted herein include stainless steel such as SUS304, a nickel titanium alloy, a cobalt chrome alloy and tungsten steel. Further, it is also possible to form a joint structure in which a plurality of the above-mentioned materials are combined. The distal tip 120 of the catheter 1 and the distal tip 220 of the light irradiation device 2 are preferably flexible and can be formed of, for example, a resin material such as polyurethane or polyurethane elastomer. The connector 140 of the catheter 1 can be formed of a resin material such as polyamide, polypropylene, polycarbonate, polyacetal, or polyether sulfone. The first sealing part 191 of the catheter 1 can be formed with an adhesive such as an epoxy adhesive or a metal solder such as silver brazing, gold brazing, zinc, Sn-Ag alloy or Au-Sn alloy.

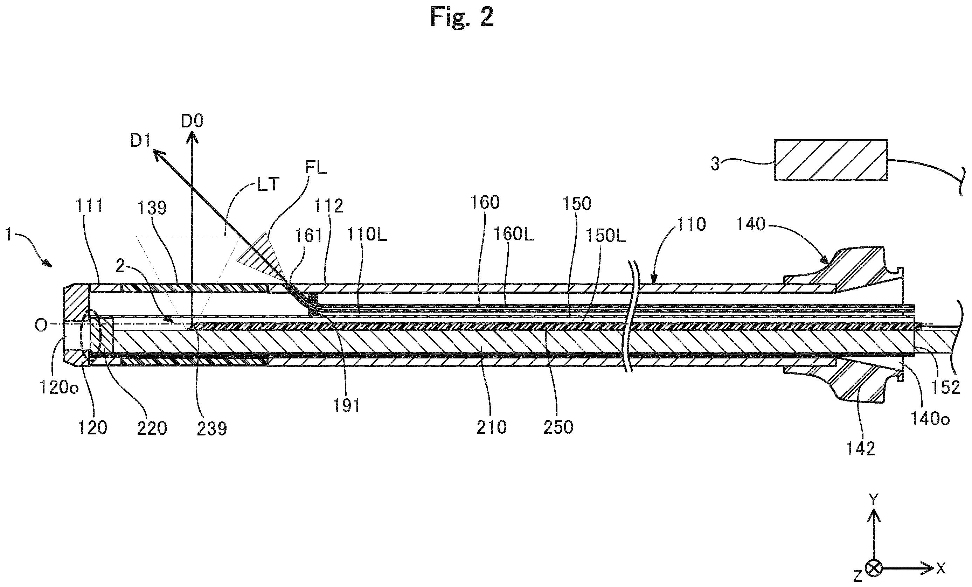

Fig. 2 is an explanatory view illustrating a use state of the light irradiation system. A method for using the light irradiation system will be described with reference to Figs. 1 and 2. First, an operator inserts a guide wire into a biological lumen. Next, the operator inserts the proximal end side of the guide wire from the opening 120o of the distal tip 120 of the catheter 1 shown in Fig. 1 into the device lumen 150L, causing the guide wire to project from the opening 152 on the proximal end side. Next, the operator pushes the catheter 1 into the biological lumen along the guide wire, so as to deliver the light emission part 139 of the catheter 1 to a target tissue of light irradiation (for example, in the case of NIR-PIT, near cancer cells). In this manner, through insertion of the guide wire into the device lumen 150L, the operator can easily deliver the catheter 1 to the target tissue in the biological lumen. The operator then withdraws the guide wire from the catheter 1.

Next, as shown in Fig. 2, the operator inserts the light irradiation device 2 from the opening 152 on the proximal end side of the catheter 1. The operator pushes the light irradiation device 2 toward the distal end side of the catheter 1 along the device lumen 150L of the catheter 1. Here, as described above, if the outer diameter Φ3 of the light irradiation device 2 is designed to be smaller than the diameter Φ2 of the device lumen 150L of the catheter 1 and larger than the diameter Φ1 of the distal tip 120 of the catheter 1, when the light irradiation device 2 is inserted into catheter 1, a distal end surface 220e of the light irradiation device 2 abuts on the inner surface of the distal chip 120, so as to be able to prevent the light irradiation device 2 from coming off to the distal end side (Fig. 2: circle frame denoted with a broken line). As shown in Fig. 2, the light irradiation part 239 of the light irradiation device 2 is arranged at a position in proximity to the light emission part 139 of the catheter 1 in a state where the distal end surface 220e of the light irradiation device 2 is abutted against the inner surface of the distal tip 120.

After that, the operator drives the laser light generator 3, so that laser light LT is irradiated by the light irradiation part 239. The light LT irradiated by the light irradiation part 239 passes through the light emission part 139 of the catheter 1 and is emitted to an external target tissue. Specifically, the light emission part 139 of the catheter 1 emits light LT from an overlapping range where the transmission range of the light LT in which the light emission part 139 of the catheter 1 is provided and the irradiation range of the light LT from the light irradiation part 239 of the light irradiation device 2 overlap each other. Hereinafter, the center of this overlapping range is also referred to as "light emission direction D0" (Fig. 2: arrow D0).

The operator releases a fluid from the first opening 161 at the same time as driving the laser light generator 3 or before and after driving the laser light generator 3. Specifically, the operator attaches a syringe containing the fluid to the opening 162 on the proximal end side of the catheter 1 and supplies the fluid from the syringe to the first fluid lumen 160L. The fluid supplied to the first fluid lumen 160L travels from the proximal end side to the distal end side of the first fluid lumen 160L, and is released to the outside from the first opening 161 communicating with the distal end part of the first fluid lumen 160L (Fig. 2: fluid FL). Hereinafter, the center of this releasing range of the fluid FL is also referred to as "fluid releasing direction D1" (Fig. 2: arrow D1).

Here, as described above, in the catheter 1 according to the present embodiment, the first opening 161 is arranged on the proximal end side beyond the light emission part 139. Further, the distal end part of the first inner shaft 160 (first fluid lumen 160L) communicating with the first opening 161 is curved from the proximal end side to the distal end side in a direction (to the outside) away from the axis O. Therefore, as shown in Fig. 2, the releasing direction D1 of the fluid from the first opening 161 is in a direction inclined toward the side of the light emission part 139. Further, the releasing direction D1 of the fluid from the first opening 161 and the light emission direction D0 from the light emission part 139 intersect in the long axis direction (axis O direction) of the catheter 1.

As described above, according to the light irradiation system of the first embodiment, the catheter 1 includes a light emission part 139 that emits the light LT in a predetermined outward direction D0 and a first opening 161 capable of releasing the fluid FL, wherein the releasing direction D1 of the fluid FL from the first opening 161 and the emission direction D0 of the light LT from the light emission part 139 intersect in the long axis direction (axis O direction) of the catheter 1 (Fig. 1). Therefore, the fluid FL can be directly supplied to the irradiation site of the light LT. As a result, for example, when physiological saline is used as the fluid FL, a body fluid at the irradiation site of the light LT can be effectively removed, and thus the concentration of the body fluid can be locally lowered. Specifically, when the catheter 1 is inserted into a blood vessel, a local decrease in blood concentration in the vicinity of the light irradiation site can suppress the light absorption by blood, and as a result, this can contribute to the suppression of the occurrence of an event that the light LT does not reach a target tissue. In addition, blood coagulation due to blood overheating can be suppressed. Suppression of blood coagulation can contribute not only to the improvement of safety, but also to the suppression of the occurrence of an event that the light LT is blocked by coagulated (or carbonized) blood so as to be unable to reach a target tissue. Further, for example, when physiological saline is used as the fluid FL, a target tissue can be cooled, and the temperature rise of the target tissue due to irradiation with the light LT can be suppressed. Further, for example, when a photosensitizer is used as the fluid FL, the efficiency of irradiating the target tissue with the light LT can be improved.

Further, according to the light irradiation system of the first embodiment, the first opening 161 as an opening capable of releasing the fluid FL is arranged on the proximal end side beyond the light emission part 139, wherein the fluid FL is released in such a manner that the releasing direction D1 of the fluid FL is inclined to the side of the light emission part 139 (Fig. 2). Therefore, through the use of the first opening 161, the fluid FL can be released from the proximal end side beyond the light emission part 139 to the light emission part 139. Further, since the positions of the light emission part 139 and the first opening 161 are different in the long axis direction (axis O direction), the degree of freedom in arranging the light emission part 139 and the first inner shaft 160 communicating with the first opening 161 in the catheter 1 can be improved.

Further, the light irradiation system according to the first embodiment includes the catheter 1 and the light irradiation device 2 to be inserted into the catheter 1, whereby when the light irradiation device 2 is inserted into the catheter 1, the light emission part 139 of the catheter 1 emits the light LT to the outside by allowing the light LT irradiated by the light irradiation part 239 of the light irradiation device 2 to pass therethrough (Fig. 2). In this way, the catheter 1 and the light irradiation device 2 are separately provided, so that the range of procedures the operator can adopt can be expanded.

### <Second Embodiment> (not according to the claims)

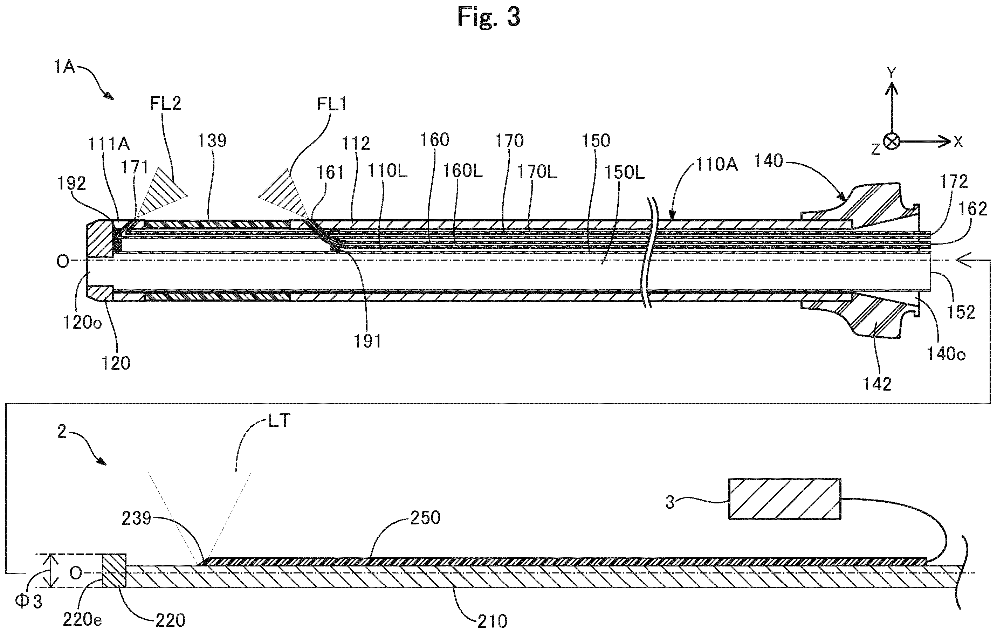

Fig. 3 is an explanatory view illustrating the configuration of the light irradiation system according to the second embodiment. The light irradiation system according to the second embodiment can further release a fluid from a second opening 171 in addition to the first opening 161. The light irradiation system according to the second embodiment includes a catheter 1A instead of the catheter 1 described in the first embodiment. In the configuration described in the first embodiment, the catheter 1A includes a main body part 110A instead of the main body part 110, and also includes a second inner shaft 170 and a second sealing part 192.

The main body part 110A includes a first shaft 111A instead of the first shaft 111. The second opening 171 bringing the inside and outside of the cylinder into communication is provided on the side of the first shaft 111A. The second opening 171 leads to a second fluid lumen 170L of the second inner shaft 170, and thus is capable of releasing the fluid supplied through the second fluid lumen 170L to the outside.

The second inner shaft 170 is a long tubular member arranged along the axis O. The second inner shaft 170 has a hollow substantially cylindrical shape having openings formed at the distal end part and the proximal end part, respectively, and having an inner cavity inside (second fluid lumen 170L) for bringing the two openings into communication. The second inner shaft 170 has substantially the same outer diameter as that of the first inner shaft 160. The second inner shaft 170 is inserted into the first shaft 111A, the light emission part 139, and the inner cavity 110L of the second shaft 112. The distal end part of the second inner shaft 170 is curved and joined to the first shaft 111A in a state where the second fluid lumen 170L and the second opening 171 communicate with each other. Joining can be realized by any method such as joining with an adhesive or joining with a metal solder. The proximal end part of the second inner shaft 170 projects from the proximal end part of the connector 140, and is configured so that the fluid can be supplied from the opening (the opening 172 on the proximal end side) of the proximal end part to the second fluid lumen 170L. Note that the proximal end part of the second inner shaft 170 may be configured so that it is assembled inside the connector 140 and the fluid can be supplied to the second fluid lumen 170L via the connector 140. The second inner shaft 170 may be formed of for example materials similar to those of the first inner shaft 160 according to the first embodiment.

The second sealing part 192 is a joint member provided at the distal end part of the second inner shaft 170 between the outer peripheral surface of the second inner shaft 170, the surface on the proximal end side of the distal tip 120, and the inner peripheral surface of the first shaft 111A. The second sealing part 192 is provided to maintain the curved shape of the distal end part of the second inner shaft 170. The second sealing part 192 can be formed of, for example materials similar to those of the first sealing part 191 according to the first embodiment.

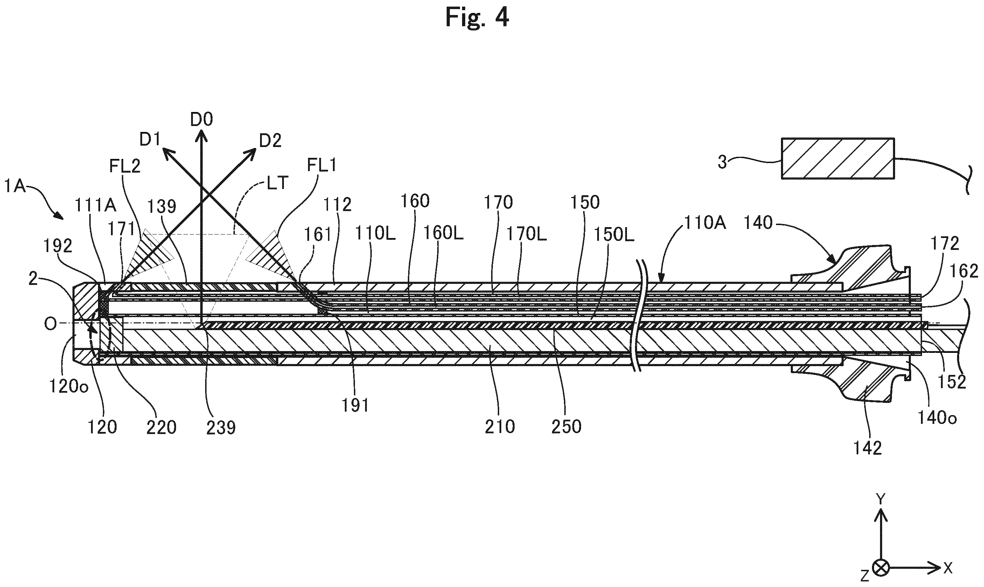

Fig. 4 is an explanatory view illustrating the use state of the light irradiation system according to the second embodiment. Upon the use of the light irradiation system according to the second embodiment, the operator releases a fluid from the second opening 171 at the same time as driving the laser light generator 3 or before and after driving the laser light generator 3. Specifically, the operator attaches a syringe containing a fluid to the opening 172 on the proximal end side of the catheter 1A and supplies the fluid from the syringe to the second fluid lumen 170L. The fluid supplied to the second fluid lumen 170L is released to the outside from the second opening 171 communicating with the distal end part of the second fluid lumen 170L (Fig. 4: fluid FL2). Hereinafter, the center of this releasing range of the fluid FL2 is also referred to as "fluid releasing direction D2" (Fig. 2: arrow D2). Note that in Fig. 4, the fluid to be released from the first opening 161 is denoted as a fluid FL1 for distinction. The fluid to be supplied to the first fluid lumen 160L and the fluid to be supplied to the second fluid lumen 170L may be the same or different.

Here, in the catheter 1A according to the second embodiment, the second opening 171 is arranged on the distal end side beyond the light emission part 139. Further, the distal end part of the second inner shaft 170 (second fluid lumen 170L) communicating with the second opening 171 is curved from the distal end side to the proximal end side in a direction (to the outside) away from the axis O. Therefore, as shown in Fig. 4, the releasing direction D2 of the fluid from the second opening 171 is in a direction inclined to the side of the light emission part 139. Further, the releasing direction D2 of the fluid from the second opening 171 and the light emission direction D0 from the light emission part 139 intersect in the long axis direction (axis O direction) of the catheter 1A.

In this way, the configuration of the catheter 1A can be variously changed and may be provided with the second opening 171 that is arranged on the distal end side beyond the light emission part 139 and capable of releasing a fluid to the outside. In the second embodiment, the catheter 1A is configured to include the second opening 171 in addition to the first opening 161 described in the first embodiment. However, the catheter 1A may also be configured to include the second opening 171 instead of the first opening 161. In this case, the first inner shaft 160 communicating with the first opening 161 can be omitted. Further, in a configuration including both the first opening 161 and the second opening 171, one inner shaft that is bifurcated and communicates with both the first opening 161 and the second opening 171, respectively, may be provided.

The light irradiation system including the catheter 1A according to the second embodiment can also exert similar effects as in the first embodiment. Further, according to the light irradiation system of the second embodiment, the second opening 171 as an opening capable of releasing the fluid FL2 is arranged on the distal end side beyond the light emission part 139, wherein the fluid FL2 is released in such a manner that the releasing direction D2 of the fluid FL2 is inclined to the side of the light emission part 139. Therefore, through the use of the second opening 171, the fluid FL2 can be released from the distal end side beyond the light emission part 139 to the light emission part 139. Further, since the positions of the light emission part 139 and the second opening 171 are different in the long axis direction, the degree of freedom in arranging the light emission part 139 and the second inner shaft 170 communicating with the second opening 171 in the catheter 1A can be improved.

### <Third Embodiment>

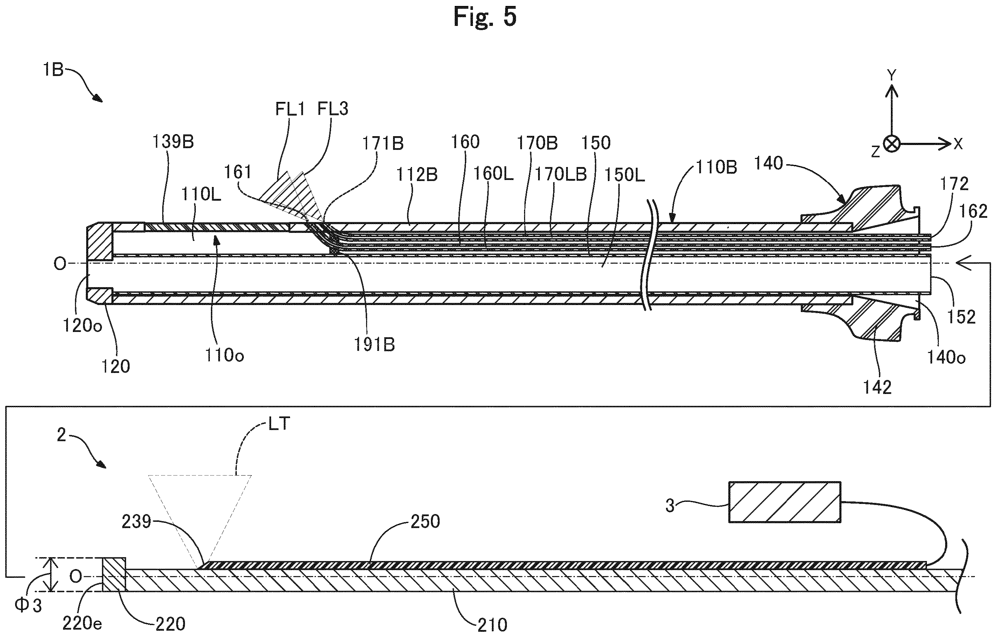

Fig. 5 is an explanatory view illustrating the configuration of the light irradiation system according to the third embodiment. The light irradiation system according to the third embodiment can further release a fluid from a third opening 171B in addition to the first opening 161. The light irradiation system according to the third embodiment includes a catheter 1B instead of the catheter 1 described in the first embodiment. In the configuration described in the first embodiment, the catheter 1B includes a main body part 110B instead of the main body part 110, and a first sealing part 191B instead of the first sealing part 191, and further includes a third inner shaft 170B.

The main body part 110B does not include the first shaft 111, but includes a second shaft 112B instead of the second shaft 112 and a light emission part 139B instead of the light emission part 139. The second shaft 112B is a hollow, substantially cylindrical member having a substantially constant outer diameter and inner diameter. The distal end part of the second shaft 112B is fixed by a distal tip 120 and the proximal end part of the second shaft 112B is fixed by the connector 140.

On a portion of the side of the second shaft 112B, two openings (opening 110o and the third opening 171B) are formed at positions different in the circumferential direction. The opening 110o is a substantially rectangular opening that communicates with the inside and outside of the second shaft 112B, and the light emission part 139 is fitted therein. The light emission part 139B is a substantially rectangular and curved member that is fitted in the opening 110o of the second shaft 112B. Similar to the first embodiment, the light emission part 139B is formed of an optically transparent resin material for allowing the light LT inside the main body part 110B to pass therethrough to the outside. The third opening 171B is a substantially circular opening that is provided on the proximal end side beyond the opening 110o and communicates with the inside and outside of the second shaft 112B. The third opening 171B leads to a third fluid lumen 170LB of the third inner shaft 170B, and thus is capable of releasing the fluid supplied through the third fluid lumen 170LB to the outside.

The third inner shaft 170B is a long tubular member arranged along the axis O. The third inner shaft 170B has a hollow substantially cylindrical shape having openings formed at the distal end part and the proximal end part, respectively, and having an inner cavity inside (third fluid lumen 170LB) for bringing the two openings into communication. The third inner shaft 170B has substantially the same outer diameter as that of the first inner shaft 160. The third inner shaft 170B is inserted into the inner cavity 110L of the second shaft 112B. The distal end part of the third inner shaft 170B is curved and joined to the second shaft 112B in a state where the third fluid lumen 170LB and the third opening 171B communicate with each other. Joining can be realized by any method. The proximal end part of the third inner shaft 170B projects from the proximal end part of the connector 140, and is configured so that a fluid can be supplied from the opening (the opening 172 on the proximal end side) of the proximal end part to the third fluid lumen 170LB. Note that the proximal end part of the third inner shaft 170B may be configured so that it is assembled inside the connector 140 and a fluid can be supplied to the third fluid lumen 170LB via the connector 140.

The first sealing part 191B is a joint member provided at both distal end parts of the first inner shaft 160 and the third inner shaft 170B, between the outer peripheral surfaces of the first inner shaft 160 and the third inner shaft 170B, and the inner peripheral surface of the second shaft 112B. The first sealing part 191B is provided to maintain the curved shape of both the distal end parts of the first inner shaft 160 and the third inner shaft 170B.

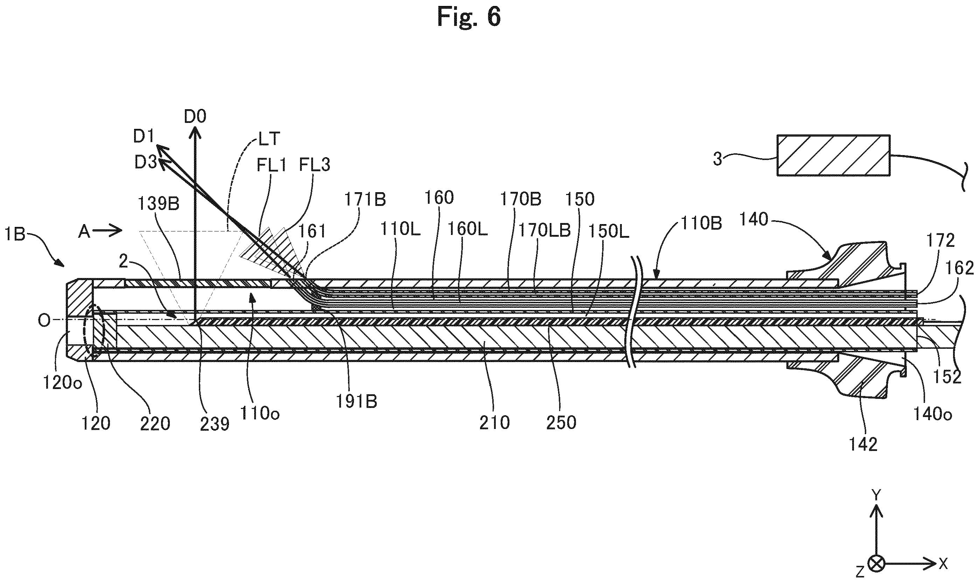

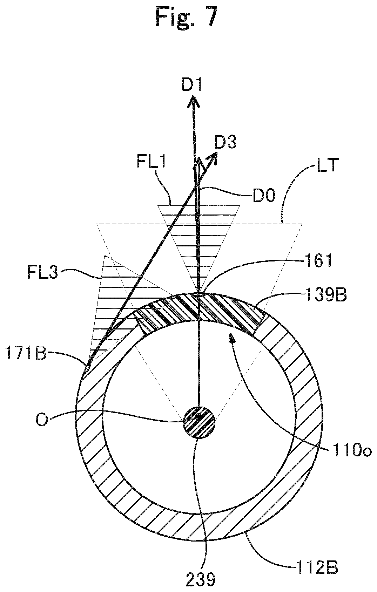

Fig. 6 is an explanatory view illustrating the use state of the light irradiation system according to the third embodiment. Fig. 7 is a schematic diagram of a light irradiation system seen from direction A in Fig. 6. Fig. 7 illustrates, for convenience of description, the position of the first opening 161 and the position of the third opening 171B overlapping one another in a sectional view of the second shaft 112B and the light emission part 139B of the catheter 1B, and the light irradiation part 239 of the light irradiation device 2.

Upon the use of the light irradiation system according to the third embodiment, the operator releases a fluid from the third opening 171B at the same time as driving the laser light generator 3 or before and after driving the laser light generator 3. Specifically, the operator attaches a syringe containing the fluid to the opening 172 on the proximal end side of the catheter 1B and supplies the fluid from the syringe to the third fluid lumen 170LB. The fluid supplied to the third fluid lumen 170LB is released to the outside from the third opening 171B communicating with the distal end part of the third fluid lumen 170LB (Fig. 6, Fig. 7: fluid FL3). Hereinafter, the center of this releasing range of the fluid FL3 is also referred to as "fluid releasing direction D3" (Fig. 6, Fig. 7: arrow D3). The fluid to be supplied to the first fluid lumen 160L and the fluid to be supplied to the third fluid lumen 170LB may be the same or different.

Here, in the catheter 1B according to the third embodiment 3, a third opening 171B and an opening 110o at which the light emission part 139B is provided are arranged at positions different in the circumferential direction. Further as shown in Fig. 7, the distal end part of the third inner shaft 170B (the third fluid lumen 170LB) communicating with the third opening 171B is curved from the outside of the light emission part 139B to the light emission part 139B in the circumferential direction of the main body part 110B. Therefore, as shown in Fig. 7, the releasing direction D3 of the fluid from the third opening 171B is in a direction inclined to the side of the light emission part 139B. Further, the releasing direction D3 of the fluid from the third opening 171B and the light emission direction D0 from the light emission part 139B intersect in the circumferential direction of the catheter 1B.

In this way, the configuration of the catheter 1B can be variously changed, and may be provided with a third opening 171B that is arranged at a position different in the circumferential direction from the light emission part 139B and capable of releasing a fluid to the outside. In the third embodiment, the catheter 1B is configured to include the third opening 171B in addition to the first opening 161 described in the first embodiment. However, the catheter 1B may also be configured to include the third opening 171B instead of the first opening 161. In this case, the first inner shaft 160 communicating with the first opening 161 can be omitted. Further, in a configuration including both the first opening 161 and the third opening 171B, one inner shaft that is bifurcated and communicates with both the first opening 161 and the third opening 171B, respectively, may be provided.

The light irradiation system including the catheter 1B according to the third embodiment can also exert similar effects as in the first embodiment. Further, according to the light irradiation system of the third embodiment, the third opening 171B as an opening capable of releasing a fluid FL3 is arranged at a position different in the circumferential direction from the position for emission of the light LT by the light emission part 139B, wherein the fluid FL3 is released in such a manner that the releasing direction D3 of the fluid FL3 is inclined to the side of the light emission part 139B. Therefore, through the use of the third opening 171B, the fluid FL3 can be released from the position different in the circumferential direction to the light emission part 139B. Further, since the positions of the light emission part 139B and the third opening 171B are different in the circumferential direction, the degree of freedom in arranging the light emission part 139B and the third inner shaft 170B communicating with the third opening 171B in the catheter 1B can be improved.

### <Fourth Embodiment> (not according to the claims)

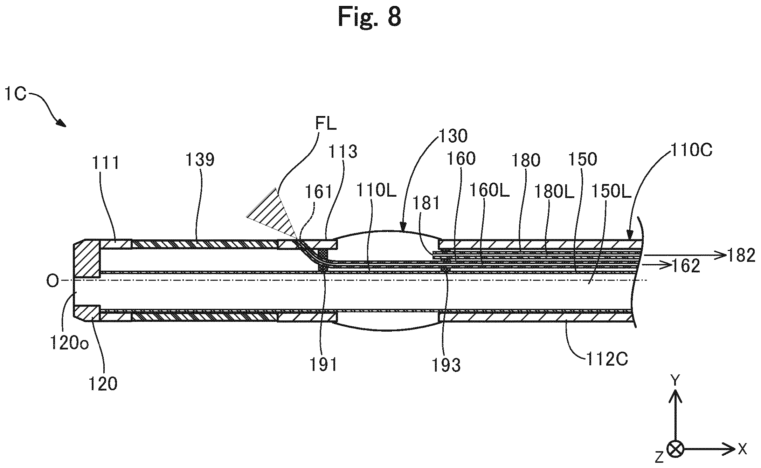

Fig. 8 is an explanatory view illustrating the configuration of the distal end side of a catheter 1C according to the fourth embodiment. The light irradiation system according to the fourth embodiment includes the catheter 1C instead of the catheter 1 described in the first embodiment. The catheter 1C includes a first expansion/contraction part 130 that is expandable and contractable in the radial direction and arranged on the proximal end side of the light emission part 139 in the configuration described in the first embodiment. Further, the catheter 1C includes a main body part 110C instead of the main body part 110 and further includes a fourth inner shaft 180 and a third sealing part 193.

The main body part 110C includes a third shaft 113 and a second shaft 112C, instead of the second shaft 112 described in the first embodiment. The third shaft 113 and the second shaft 112C are both hollow, substantially cylindrical members, having substantially constant outer diameters and inner diameters. The distal end part of the third shaft 113 is joined to the proximal end part of the light emission part 139 and the distal end part of the first expansion/contraction part 130 is joined to the proximal end part of the third shaft 113. The proximal end part of the first expansion/contraction part 130 is joined to the distal end part of the second shaft 112C and the proximal end part of the second shaft 112 is fixed by the connector 140. The first opening 161 described in the first embodiment is provided on the side of the third shaft 113.

The first expansion/contraction part 130 is expandable and contractable in the radial direction (YZ-axis direction), and is a tubular balloon member having both ends on the distal end side and the proximal end side, which are open. The first expansion/contraction part 130 performs out-of-plane deformation when an expansion medium is supplied to the interior (inside) to expand (increasing the diameter) outwardly in the radial direction. The first expansion/contraction part 130 performs in-plane deformation when an expansion medium is discharged to contract (decreasing the diameter) inwardly in the radial direction. In this way, the first expansion/contraction part 130 is freely expandable/contractable through the supply/discharge of the expansion medium. The expansion pressure, outer diameter, and length of the first expansion/contraction part 130 can be arbitrarily determined. Note that as the expansion medium, for example, a liquid containing a contrast medium having radiopacity, physiological saline, or the like can be used.

The first expansion/contraction part 130 is expandable and contractable with the internal pressure changes, and is formed of materials having flexibility with which damages within blood vessels can be suppressed and hardness which allows expansion within lesions. For example, the first expansion/contraction part 130 can be formed of polyolefins such as polyethylene, polypropylene, and ethylene-propylene copolymers, polyesters such as polyethylene terephthalate, thermoplastic resins such as polyvinyl chloride, ethylene-vinyl acetate copolymers, crosslinked ethylene-vinyl acetate copolymers, and polyurethanes, polyamide elastomer, polyolefin elastomer, silicone rubber, latex rubber, or the like.

The fourth inner shaft 180 is a long tubular member arranged along the axis O. Specifically, the fourth inner shaft 180 has a hollow substantially cylindrical shape having openings formed at the distal end part and the proximal end part, respectively, and having an inner cavity inside (first fluid lumen 180L) for bringing the two openings into communication. The fourth inner shaft 180 has a smaller diameter than that of the second shaft 112C and is inserted into the inner cavity 110L of the second shaft 112C. The distal end part of the fourth inner shaft 180 projects from the distal end part of the second shaft 112C and is located within the first expansion/contraction part 130. The proximal end part of the fourth inner shaft 180 projects from the proximal end part of the connector 140, and is configured so that the expansion medium can be supplied by the operator. The proximal end part of the fourth inner shaft 180 may be configured so that it is assembled inside the connector 140 and the fluid can be supplied to the expansion medium lumen 180L via the connector 140. The fourth inner shaft 180 can be formed of, for example materials similar to those of the first inner shaft 160 according to the first embodiment.

The third sealing part 193 is arranged between the inner peripheral surface of the second shaft 112C and the outer peripheral surfaces of the inner shaft 150, the first inner shaft 160 and the fourth inner shaft 180, sealing the inside of the first expansion/contraction part 130. The third sealing part 193 can be formed of, for example materials similar to those of the first sealing part 191 described in the first embodiment.

When the light irradiation system according to the fourth embodiment is used, an operator expands the first expansion/contraction part 130 after delivering the light emission part 139 to a position in the vicinity of a target tissue. Specifically, the operator attaches a syringe containing the expansion medium to the opening 182 on the proximal end side of the catheter 1C and supplies a fluid from the syringe to the expansion medium lumen 180L. The fluid supplied to the expansion medium lumen 180L fills the inside of the first expansion/contraction part 130 via the fourth opening 181 communicating with the distal end part of the expansion medium lumen 180L. This causes the first expansion/contraction part 130 to be in an expanded state. Thereafter, the operator may drive the laser light generator 3 and release a fluid from the first opening 161 as described in the first embodiment.

In this way, the configuration of the catheter 1C can be variously changed and may include the first expansion/contraction part 130 arranged on the proximal end side beyond the first opening 161. The light irradiation system including the catheter 1C according to the fourth embodiment can also exert similar effects as in the first embodiment. Further, according to the light irradiation system of the fourth embodiment, the catheter 1C includes the first expansion/contraction part 130 that is expandable and contractable in the radial direction. Therefore, expanding the first expansion/contraction part 130 can inhibit the flow of a body fluid in a biological lumen, and can efficiently achieve a decrease in the local concentration of the body fluid due to the release of the fluid FL. Further, expanding the first expansion/contraction part 130 can position (fix) the distal end part of the catheter 1C in the biological lumen.

### <Fifth Embodiment> (not according to the claims)

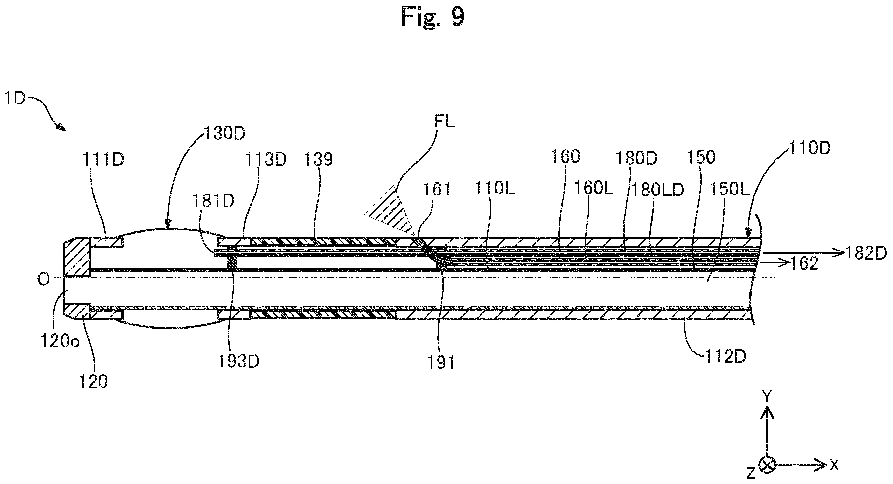

Fig. 9 is an explanatory view illustrating the configuration of the distal end side of a catheter 1D according to the fifth embodiment. The light irradiation system according to the fifth embodiment includes the catheter 1D instead of the catheter 1 described in the first embodiment. The catheter 1D includes a second expansion/contraction part 130D that is expandable and contractable in the radial direction, which is arranged on the distal end side of the light emission part 139 in the configuration described in the first embodiment. Further, the catheter 1D includes a main body part 110D instead of the main body part 110 and further includes a fourth inner shaft 180D and a third sealing part 193D.

In the main body part 110D, the distal end part of the second expansion/contraction part 130D is joined to the proximal end part of the first shaft 111D. The proximal end part of the second expansion/contraction part 130D is joined to the distal end part of the third shaft 113D, and the proximal end part of the third shaft 113D is joined to the distal end part of the light emission part 139. The distal end part of the second shaft 112D is joined to the proximal end part of the light emission part 139 and the proximal end part of the second shaft 112D is fixed by the connector 140. The first opening 161 described in the first embodiment is formed on the side of the second shaft 112D.

The second expansion/contraction part 130D has a configuration similar to that of the first expansion/contraction part 130 according to the fourth embodiment except that the arrangement in the axis O direction is different. The fourth inner shaft 180D has a configuration similar to that of the fourth inner shaft 180 according to the fourth embodiment except that the distal end part projects from the distal end part of the third shaft 113D and it is arranged within the second expansion/contraction part 130D. The third sealing part 193D is arranged between the inner peripheral surface of the third shaft 113D and the outer peripheral surfaces of the inner shaft 150 and the fourth inner shaft 180D, sealing the inside of the second expansion/contraction part 130D.

A method for using the light irradiation system according to the fifth embodiment is similar to that of the fourth embodiment. In this way, the configuration of the catheter 1D can be variously changed and may include a second expansion/contraction part 130D arranged on the distal end side beyond the light emission part 139. The light irradiation system including the catheter 1D according to the fifth embodiment can also exert similar effects as in the first embodiment. Further, according to the light irradiation system of the fifth embodiment, the catheter 1D includes the second expansion/contraction part 130D that is expandable and contractable in the radial direction. Therefore, expanding the second expansion/contraction part 130D can inhibit the flow of a body fluid in a biological lumen, and can efficiently achieve a decrease in the local concentration of the body fluid due to the release of the fluid FL. Further, expanding the second expansion/contraction part 130D can position (fix) the distal end part of the catheter 1D in the biological lumen.

### <Sixth Embodiment> (not according to the claims)

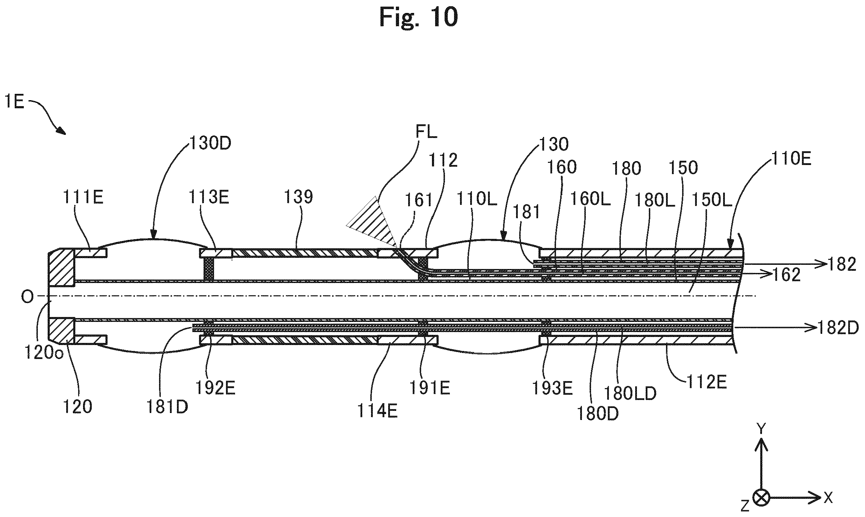

Fig. 10 is an explanatory view illustrating the configuration of the distal end side of a catheter 1E according to a sixth embodiment. The light irradiation system according to the sixth embodiment includes the catheter 1E instead of the catheter 1 described in the first embodiment. The catheter 1E includes, in the configuration described in the first embodiment, the first expansion/contraction part 130 that is expandable and contractable in the radial direction and arranged on the proximal end side of the first opening 161, and the second expansion/contraction part 130D that is expandable and contractable in the radial direction and arranged on the distal end side of the light emission part 139. Further, the catheter 1E includes a main body part 110E instead of the main body part 110 and further includes a first sealing part 191E, a second sealing part 192E, and a third sealing part 193E.

The configurations of the first expansion/contraction part 130 and the fourth inner shaft 180 for supplying an expansion medium to the first expansion/contraction part 130 are as described in the fourth embodiment. The configurations of the second expansion/contraction part 130D and the fourth inner shaft 180D for supplying an expansion medium to the second expansion/contraction part 130D are as described in the fifth embodiment.

In the main body part 110E, the distal end part of the second expansion/contraction part 130D is joined to the proximal end part of the first shaft 111E. The proximal end part of the second expansion/contraction part 130D is joined to the distal end part of the third shaft 113E, and the proximal end part of the third shaft 113E is joined to the distal end part of the light emission part 139. The distal end part of the fourth shaft 114E is joined to the proximal end part of the light emission part 139, and the distal end part of the first expansion/contraction part 130 is joined to the proximal end part of the fourth shaft 114. The proximal end part of the first expansion/contraction part 130 is joined to the distal end part of the second shaft 112E and the proximal end part of the second shaft 112E is fixed by the connector 140.

The first sealing part 191E is provided between the inner peripheral surface of the fourth shaft 114E and the outer peripheral surfaces of the inner shaft 150, the first inner shaft 160, the fourth inner shaft 180, and the fourth inner shaft 180D, sealing the inside of the first expansion/contraction part 130. Similarly, the third sealing part 193E is provided between the inner peripheral surface of the second shaft 112E and the outer peripheral surfaces of the inner shaft 150, the first inner shaft 160, the fourth inner shaft 180, and the fourth inner shaft 180D, sealing the inside of the first expansion/contraction part 130. The second sealing part 192E is provided between the inner peripheral surface of the third shaft 113E and the outer peripheral surfaces of the inner shaft 150 and the fourth inner shaft 180D, sealing the inside of the second expansion/contraction part 130D.

A method for using the light irradiation system according to the sixth embodiment is similar to that of the fourth and the fifth embodiments. In this way, the configuration of the catheter 1E can be variously changed and may include both the first expansion/contraction part 130 arranged on the proximal end side beyond the first opening 161 and the second expansion/contraction part 130D arranged on the distal end side beyond the light emission part 139. Such light irradiation system including the catheter 1E according to the sixth embodiment can also exert similar effects as in the first embodiment. Further, according to the light irradiation system according to the sixth embodiment, the first opening 161 and the light emission part 139 are provided between the first expansion/contraction part 130 and the second expansion/contraction part 130D (Fig. 10). Therefore, expanding the first expansion/contraction part 130 and the second expansion/contraction part 130D can lower the concentration of a body fluid in the vicinity of the light emission part 139 (between the first expansion/contraction part 130 and the second expansion/contraction part 130D) more efficiently, and thus can further improve the efficiency of irradiating a target tissue with light.

### <Seventh Embodiment> (not according to the claims)

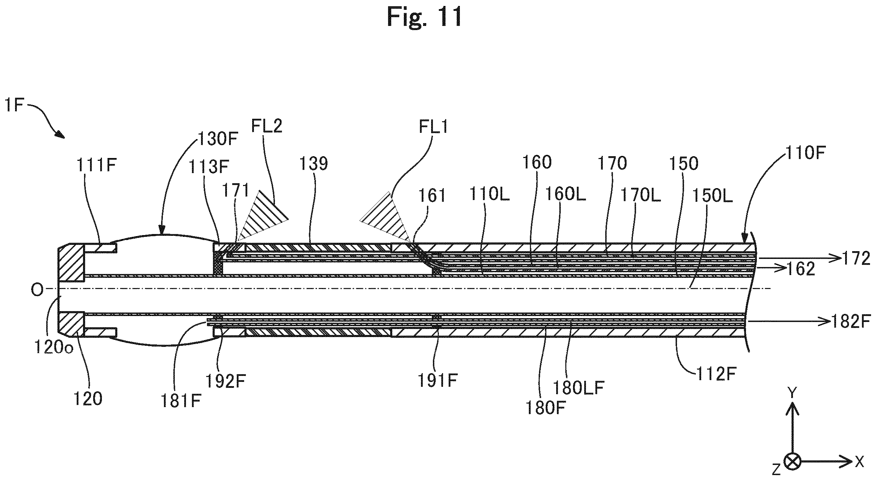

Fig. 11 is an explanatory view illustrating the configuration of the distal end side of a catheter 1F according to the seventh embodiment. The light irradiation system according to the seventh embodiment includes the catheter 1F instead of the catheter 1A described in the second embodiment. The catheter 1F includes a third expansion/contraction part 130F that is expandable and contractable in the radial direction, and is arranged on the distal end side of the second opening 171. In the configuration described in the second embodiment, the catheter 1F includes a main body part 110F instead of the main body part 110A, a first sealing part 191F instead of the first sealing part 191, and a second sealing part 192F instead of the second sealing part 192, respectively, and further includes a fourth inner shaft 180F.

The configurations of the third expansion/contraction part 130F, and, the fourth inner shaft 180F for supplying an expansion medium to the third expansion/contraction part 130F are as described in the fifth embodiment. In the main body part 110F, the distal end part of the third expansion/contraction part 130F is joined to the proximal end part of the first shaft 111F. The proximal end part of the third expansion/contraction part 130F is joined to the distal end part of the third shaft 113F, and the proximal end part of the third shaft 113F is joined to the distal end part of the light emission part 139. The distal end part of the second shaft 112F is joined to the proximal end part of the light emission part 139 and the proximal end part of the second shaft 112F is fixed by the connector 140.

The first sealing part 191F is provided between the inner peripheral surface of the second shaft 112F and the outer peripheral surfaces of the inner shaft 150, the first inner shaft 160, the second inner shaft 170, and the fourth inner shaft 180F to join them. The second sealing part 192F is provided between the inner peripheral surface of the third shaft 113F and the outer peripheral surfaces of the inner shaft 150, the second inner shaft 170 and the fourth inner shaft 180F, sealing the inside of the third expansion/contraction part 130F.

A method for using the light irradiation system according to the seventh embodiment is similar to that of the second and the fifth embodiments. In this way, the configuration of the catheter 1F can be variously changed and may include a third expansion/contraction part 130F arranged on the distal end side beyond the second opening 171. The light irradiation system including the catheter 1F according to the seventh embodiment can also exert similar effects as in the first and the second embodiments. Further, according to the light irradiation system of the seventh embodiment, the catheter 1F includes the third expansion/contraction part 130F that is expandable and contractable in the radial direction. Therefore, expanding the third expansion/contraction part 130F can inhibit the flow of a body fluid in a biological lumen, and can efficiently achieve a decrease in the local concentration of the body fluid due to the release of the fluid FL1 and the fluid FL2. Further, expanding the third expansion/contraction part 130F can position (fix) the distal end part of the catheter 1F in the biological lumen.

### <Eighth Embodiment> (not according to the claims)

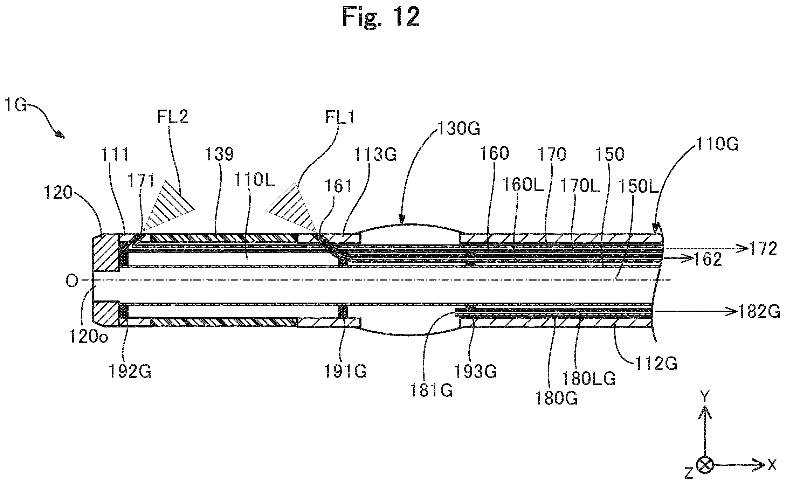

Fig. 12 is an explanatory view illustrating the configuration of the distal end side of a catheter 1G according to the eighth embodiment. The light irradiation system according to the eighth embodiment includes the catheter 1G instead of the catheter 1A described in the second embodiment. The catheter 1G includes a fourth expansion/contraction part 130G that is expandable and contractable in the radial direction, and arranged on the proximal end side of the light emission part 139. In the configuration described in the second embodiment, the catheter 1G includes a main body part 110G instead of the main body part 110A, a first sealing part 191G instead of the first sealing part 191, and a second sealing part 192G instead of the second sealing part 192, respectively, and further includes a fourth inner shaft 180G and a third sealing part 193G.

The configurations of the fourth expansion/contraction part 130G, and, the fourth inner shaft 180G for supplying an expansion medium to the fourth expansion/contraction part 130G are as described in the fourth embodiment. In the main body part 110G, the distal end part of the third shaft 113G is joined to the proximal end part of the light emission part 139, and the distal end part of the fourth expansion/contraction part 130G is joined to the proximal end part of the third shaft 113G. The proximal end part of the fourth expansion/contraction part 130G is joined to the distal end part of the second shaft 112G and the proximal end part of the second shaft 112G is fixed by the connector 140.

The first sealing part 191G is provided between the inner surface of the third shaft 113G and the outer peripheral surfaces of the inner shaft 150, the first inner shaft 160 and the second inner shaft 170, sealing the inside of the fourth expansion/contraction part 130G. Similarly, the third sealing part 193G is provided between the inner surface of the second shaft 112G and the outer peripheral surfaces of the inner shaft 150, the first inner shaft 160 and the second inner shaft 170, sealing the inside of the fourth expansion/contraction part 130G. The second sealing part 192G is provided between the inner surface of the first shaft 111 and the outer peripheral surfaces of the inner shaft 150 and the second inner shaft 170 to join them.

A method for using the light irradiation system according to the eighth embodiment is similar to that of the second and the fourth embodiments. In this way, the configuration of the catheter 1G can be variously changed and may include a fourth expansion/contraction part 130G arranged on the proximal end side beyond the light emission part 139. The light irradiation system including the catheter 1G according to the eighth embodiment can also exert similar effects as in the first and the second embodiments. Further, according to the light irradiation system of the eighth embodiment, the catheter 1G includes the fourth expansion/contraction part 130G that is expandable and contractable in the radial direction. Therefore, expanding the fourth expansion/contraction part 130G can inhibit the flow of a body fluid in a biological lumen, and can efficiently achieve a decrease in the local concentration of the body fluid due to the release of the fluid FL1 and the fluid FL2. Further, expanding the fourth expansion/contraction part 130G can position (fix) the distal end part of the catheter 1G in the biological lumen.

Further, the catheter 1G may also include the third expansion/contraction part 130F described in the seventh embodiment in addition to the fourth expansion/contraction part 130G. In this case, the second opening 171 and the light emission part 139 are provided between the third expansion/contraction part 130F and the fourth expansion/contraction part 130G. Therefore, expanding the third expansion/contraction part 130F and the fourth expansion/contraction part 130G can lower the concentration of a body fluid in the vicinity of the light emission part 139 (between the third expansion/contraction part 130F and the fourth expansion/contraction part 130G) more efficiently, and thus can further improve the efficiency of irradiating a target tissue with light LT.

### <Ninth embodiment>

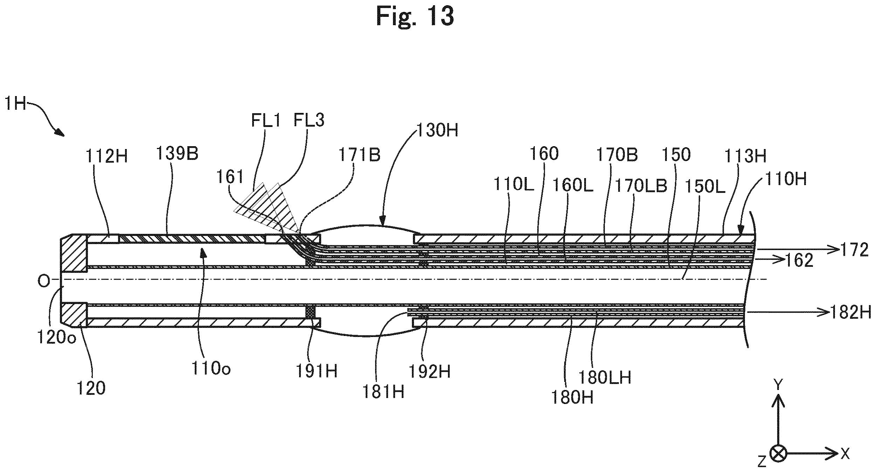

Fig. 13 is an explanatory view illustrating the configuration of the distal end side of a catheter 1H according to a ninth embodiment. The light irradiation system according to the ninth embodiment includes the catheter 1H instead of the catheter 1B described in the third embodiment. The catheter 1H includes a fifth expansion/contraction part 130H that is expandable and contractable in the radial direction, and is arranged on the proximal end side of the third opening 171B. In the configuration described in the third embodiment, the catheter 1H includes a main body part 110H instead of the main body part 110B and a first sealing part 191H instead of the first sealing part 191, respectively, and further includes a fourth inner shaft 180H and a second sealing part 192H.

The configurations of the fifth expansion/contraction part 130H, and, the fourth inner shaft 180H for supplying an expansion medium to the fifth expansion/contraction part 130H are as described in the fourth embodiment. In the main body part 110H, the fifth expansion/contraction part 130H is joined to the proximal end part of the second shaft 112H where the light emission part 139B is formed. A hollow, substantially cylindrical third shaft 113H having substantially constant outer diameter and inner diameter is arranged on the proximal end side of the second shaft 112H. The fifth expansion/contraction part 130H is joined to the distal end part of the third shaft 113H and the proximal end part of the third shaft 113H is fixed by the connector 140.

The first sealing part 191H is provided between the inner peripheral surface of the second shaft 112H and the outer peripheral surfaces of the inner shaft 150, the first inner shaft 160 and the second inner shaft 170, sealing the inside of the fifth expansion/contraction part 130H. Similarly, the second sealing part 192H is provided between the inner peripheral surface of the third shaft 113H and the outer peripheral surfaces of the inner shaft 150, the first inner shaft 160, the second inner shaft 170, and the fourth inner shaft 180H, sealing the inside of the fifth expansion/contraction part 130H.

A method for using the light irradiation system according to the ninth embodiment is similar to that of the third and the fourth embodiments. In this way, the configuration of the catheter 1H can be variously changed and may include a fifth expansion/contraction part 130H arranged on the proximal end side beyond the third opening 171B. The light irradiation system including the catheter 1H according to the ninth embodiment can also exert similar effects as in the first and the third embodiments. Further, according to the light irradiation system of the ninth embodiment, the catheter 1H includes the fifth expansion/contraction part 130H that is expandable and contractable in the radial direction. Therefore, expanding the fifth expansion/contraction part 130H can inhibit the flow of a body fluid in a biological lumen, and can efficiently achieve a decrease in the local concentration of the body fluid due to the release of the fluid FL1 and the fluid FL3. Further, expanding the fifth expansion/contraction part 130H can position (fix) the distal end part of the catheter 1H in the biological lumen.

### <Tenth Embodiment>

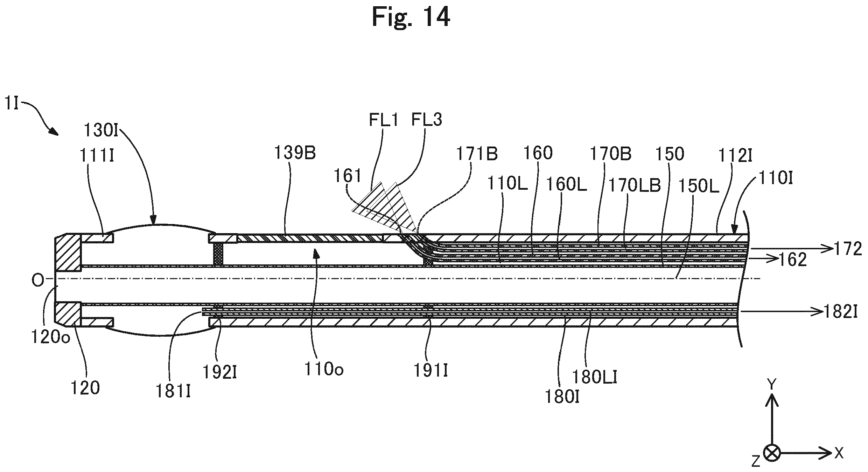

Fig. 14 is an explanatory view illustrating the configuration of the distal end side of a catheter 1I according to the tenth embodiment. The light irradiation system according to the tenth embodiment includes the catheter 1I instead of the catheter 1B described in the third embodiment. The catheter 1I includes a sixth expansion/contraction part 130I that is expandable and contractable in the radial direction, and arranged on the distal end side of the light emission part 139B. In the configuration described in the third embodiment, the catheter 1I includes a main body part 110I instead of the main body part 110B and a first sealing part 191I instead of the first sealing part 191, respectively, and further includes a fourth inner shaft 180I and a second sealing part 192I.

The configurations of the sixth expansion/contraction part 130I, and, the fourth inner shaft 180I for supplying an expansion medium to the sixth expansion/contraction part 130I are as described in the fifth embodiment. In the main body part 110I, the distal end part of the first shaft 111I is fixed by the distal tip 120, and the distal end part of the sixth expansion/contraction part 130I is joined to the proximal end part of the first shaft 111I. Further, the proximal end part of the sixth expansion/contraction part 130I is joined to the distal end part of the second shaft 112I where the light emission part 139B is formed. The proximal end part of the second shaft 112I is fixed by the connector 140.

The first sealing part 191I is provided between the inner peripheral surface of the second shaft 112I and the outer peripheral surfaces of the inner shaft 150, the first inner shaft 160, the third inner shaft 170B, and the fourth inner shaft 180I to join them. The second sealing part 192I is provided between the inner peripheral surface of the second shaft 112I and the outer peripheral surfaces of the inner shaft 150, the first inner shaft 160, the third inner shaft 170B, and the fourth inner shaft 180I, sealing the inside of the sixth expansion/contraction part 130I.

A method for using the light irradiation system according to the tenth embodiment is similar to that of the third and the fifth embodiments. In this way, the configuration of the catheter 1I can be variously changed and may include a sixth expansion/contraction part 130I arranged on the distal end side beyond the light emission part 139B. The light irradiation system including the catheter 1I according to the tenth embodiment can also exert similar effects as in the first and the third embodiments. Further, according to the light irradiation system of the tenth embodiment, the catheter 1I includes the sixth expansion/contraction part 130I that is expandable and contractable in the radial direction. Therefore, expanding the sixth expansion/contraction part 130I can inhibit the flow of a body fluid in a biological lumen, and can efficiently achieve a decrease in the local concentration of the body fluid due to the release of the fluid FL1 and the fluid FL3. Further, expanding the sixth expansion/contraction part 130I can position (fix) the distal end part of the catheter 1I in the biological lumen.

Further, the catheter 1I may also include the fifth expansion/contraction part 130H described in the ninth embodiment in addition to the sixth expansion/contraction part 130I. In this case, the third opening 171B and the light emission part 139B are provided between the fifth expansion/contraction part 130H and the sixth expansion/contraction part 130I. Therefore, expanding the fifth expansion/contraction part 130H and the sixth expansion/contraction part 130I can lower the concentration of a body fluid in the vicinity of the light emission part 139B (between the fifth expansion/contraction part 130H and the sixth expansion/contraction part 130I) more efficiently, and thus can further improve the efficiency of irradiating a target tissue with light LT.

### <Eleventh Embodiment> (not according to the claims)

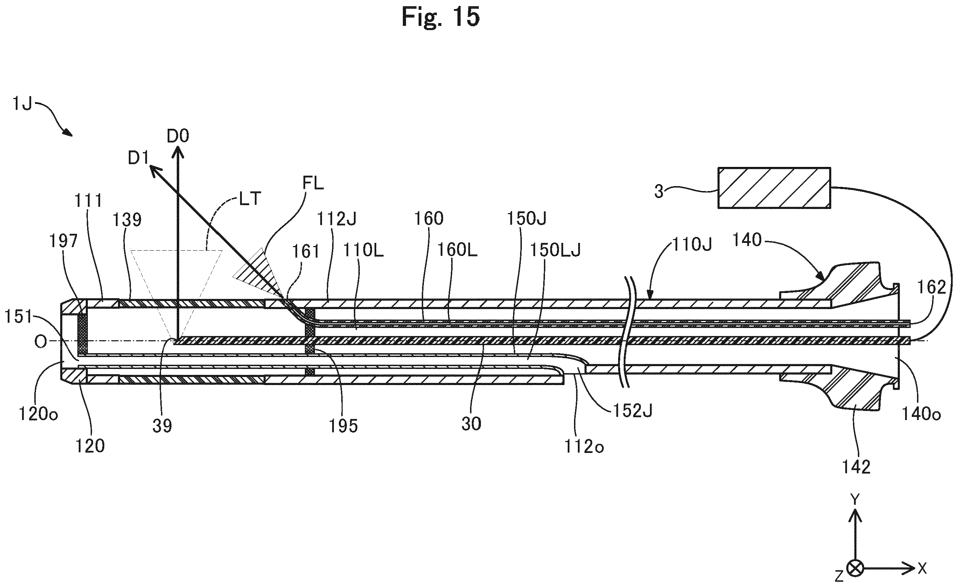

Fig. 15 is an explanatory view illustrating the configuration of the light irradiation system according to the eleventh embodiment. The light irradiation system according to the eleventh embodiment is configured of only a catheter 1J and does not include the light irradiation device 2 described in the first embodiment. The catheter 1J includes, in the catheter 1 described in the first embodiment, a main body part 110J instead of the main body part 110 and an inner shaft 150J instead of the inner shaft 150, respectively, and further includes a light transmission part 30, a light irradiation part 39, a fifth sealing part 195, and a seventh sealing part 197.

In the main body part 110J, the second shaft 112J has a configuration similar to that of the second shaft 112 according to the first embodiment except that an opening 112o communicating with the inside and outside of the cylinder on the side is formed. The opening 112o leads to a device lumen 150LJ of the inner shaft 150J, and functions as a port for taking a medical device such as a guide wire in and out of the device lumen 150LJ. The position, size, shape and the like of the opening 112o in the second shaft 112J can be arbitrarily determined.

The inner shaft 150J is a long tubular member arranged along the axis O. The inner shaft 150J has a hollow substantially cylindrical shape having openings (openings 151 and 152J) formed at the distal end part and the proximal end part, respectively, and having an inner cavity inside (device lumen 150LJ) for bringing the two openings into communication. The inner shaft 150J has a smaller diameter than the first and second shafts 111 and 112J, and is inserted into the inner cavity 110L of the first shaft 111, the light emission part 139 and the second shaft 112J. The distal end part of the inner shaft 150J is joined to the distal tip 120 by the seventh sealing part 197. The proximal end part of the inner shaft 150J is joined to the inner surface of the second shaft 112J in a state where the device lumen 150LJ and the opening 112o communicate with each other. Accordingly, the catheter 1J can be configured as namely the rapid exchange type, whereby a guide wire or the like can be inserted from the opening 151 on the side of the distal tip 120 into the device lumen 150LJ and then withdrawn from the opening 112o of the second shaft 112J to the outside. Note that joining can be realized by any method.

The light transmission part 30 is an optical fiber extending along the axis O direction from the substantially central portion of the light emission part 139 to the proximal end part of the second shaft 112J. The proximal end part of the light transmission part 30 is directly or indirectly connected via another optical fiber to the laser light generator 3 that generates laser light with an arbitrary wavelength via a connector. The laser light generator 3 functions as a "light source" installed outside the catheter 1J. The light irradiation part 39 is provided at the distal end part of the light transmission part 30, and performs irradiation with laser light LT (light LT, emitted light LT) transmitted by the light transmission part 30 in a predetermined outward direction (Fig. 15: light LT denoted with a broken line). The light irradiation part 39 can be formed by subjecting the distal end part of the light transmission part 30 to various processes in a manner similar to that for the light irradiation part 239 according to the first embodiment. The fifth sealing part 195 is a joint member provided at the distal end part of the second inner shaft 112J between the inner peripheral surface of the second shaft 112J and the outer peripheral surfaces of the inner shaft 150J, the first inner shaft 160, and the light transmission part 30.

A method for using the light irradiation system (catheter 1J) according to the eleventh embodiment is described. First, an operator inserts a guide wire into a biological lumen. Next, the operator inserts the proximal end side of the guide wire from the opening 120o of the distal tip 120 of the catheter 1J shown in Fig. 15 into the device lumen 150LJ and then withdrawing it through the opening 112o to the outside. Next, the operator pushes the catheter 1J into the biological lumen along the guide wire, so as to deliver the light emission part 139 of the catheter 1J to a target tissue of light irradiation. After that, the operator drives the laser light generator 3 to perform irradiation with laser light LT from the light irradiation part 39. The light LT irradiated by the light irradiation part 39 passes through the light emission part 139 of the catheter 1J and is then emitted to an external target tissue. Specifically, in a manner similar to that of the first embodiment, the light emission part 139 of the catheter 1J emits light LT from an overlapping range where the transmission range of the light LT in which the light emission part 139 is provided and the irradiation range of the light LT from the light irradiation part 39 overlap each other. The operator releases a fluid from the first opening 161 at the same time as driving the laser light generator 3 or before and after driving the laser light generator 3. Detailed descriptions for the method are similar to those of the first embodiment.

In this way, the configuration of the catheter 1J can be variously changed and may be configured to include the light transmission part 30 and the light irradiation part 39, and to be able to perform irradiation with the light LT by itself without the use of the light irradiation device 2 described in the first embodiment. Further, the catheter 1J according to the eleventh embodiment may also include the second opening 171 described in the second embodiment, the third opening 171B described in the third embodiment, and the first to the sixth expansion/contraction parts 130 to 130I described in the fourth to the tenth embodiments. The catheter 1J (light irradiation system) according to the eleventh embodiment can also exert similar effects as in the first embodiment.

### <Twelfth Embodiment> (not according to the claims)

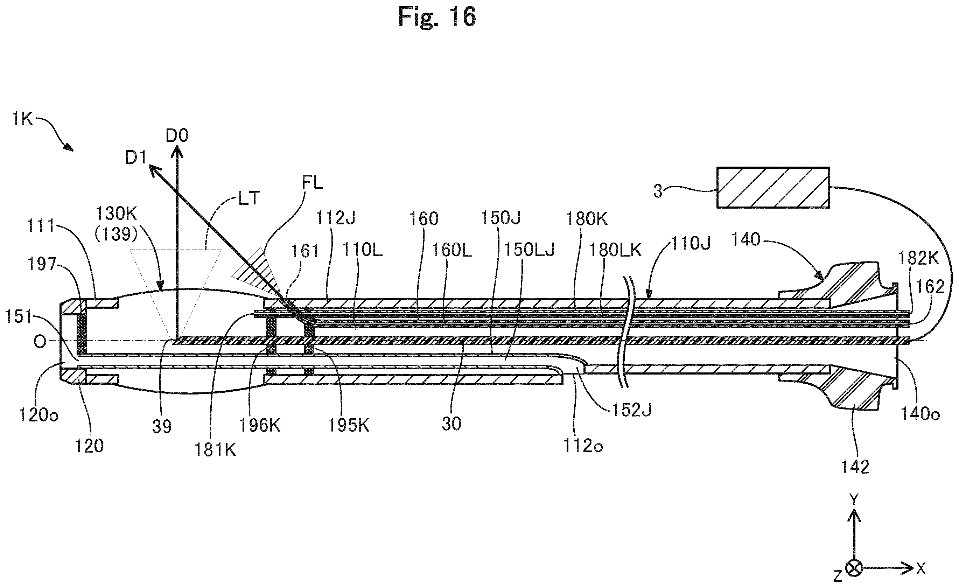

Fig. 16 is an explanatory view illustrating the configuration of the light irradiation system according to the twelfth embodiment. The light irradiation system according to the twelfth embodiment is configured of only a catheter 1K and does not include the light irradiation device 2 described in the first embodiment. The catheter 1K includes, in the catheter 1J described in the eleventh embodiment, an expansion/contraction part 130K instead of the light emission part 139 and a fifth sealing part 195K instead of the fifth sealing part 195, respectively, and further includes a fourth inner shaft 180K and a sixth sealing part 196K.

The expansion/contraction part 130K has a configuration similar to that of the first expansion/contraction part 130 described in the fourth embodiment, except that it is formed of an optically transparent resin material. The fourth inner shaft 180K has a configuration similar to that of the fourth inner shaft 180 described in the fourth embodiment except that the distal end part projects from the second shaft 112J and it is located within the expansion/contraction part 130K. The fifth sealing part 195K is a joint member provided between the inner peripheral surface of the second shaft 112J and the outer peripheral surfaces of the inner shaft 150J, the first inner shaft 160, the light transmission part 30, and the fourth inner shaft 180K. The sixth sealing part 196K is arranged between the inner peripheral surface of the second shaft 112J and the outer peripheral surfaces of the inner shaft 150J, the first inner shaft 160, the light transmission part 30, and the fourth inner shaft 180K, sealing the inside of the expansion/contraction part 130K.

A method for using the light irradiation system (catheter 1K) according to the twelfth embodiment is almost similar to the method for using the light irradiation system (catheter 1J) according to the eleventh embodiment. In the case of the catheter 1K, an operator expands the expansion/contraction part 130K after delivering the expansion/contraction part 130K to a position in the vicinity of a target tissue. Accordingly, the distal end part of the catheter 1K can be positioned (fixed) in the biological lumen. After that, the operator drives the laser light generator 3 to perform irradiation with laser light LT from the light irradiation part 39. The light LT irradiated by the light irradiation part 39 passes through the expansion/contraction part 130K of the catheter 1K and is then emitted to an external target tissue. Specifically, in the twelfth embodiment, the expansion/contraction part 130K also functions as the light emission part 139. Further, the operator releases a fluid from the first opening 161 at the same time as driving the laser light generator 3 or before and after driving the laser light generator 3. Detailed descriptions for the method are similar to those of the first embodiment.

### <Modification examples of the embodiments>

The disclosed embodiments are not limited to the above-described embodiments, and can be implemented in various aspects without departing from the gist thereof. For example, the following modification examples are also possible.

### [Modification example 1]

The first to the twelfth embodiment above describe examples of the configurations of the catheters 1 and 1A to 1K and the light irradiation device 2. However, the configurations of the catheters and the light irradiation device can be variously changed. For example, in the main body parts (first to fourth shafts) of the catheters and the main body part of the light irradiation device, braided bodies and reinforcing layers made of a coil body may be embedded. In such a manner, torquability and ability to maintain the shape can be improved. For example, the outer surface of the catheters and the outer surface of the light irradiation device may be coated with a hydrophilic or hydrophobic resin. In such a manner, slidability in biological lumens and slidability within device lumens can be improved. Further, the outer surface of the catheters and the outer surface of a balloon member may be coated with an antithrombogenic material such as heparin. This can suppress a decreased laser output due to the adhesion of blood clots to the inner and outer surfaces of the catheter as a result of light (laser light) irradiation.

For example, at least one of the catheters and the light irradiation system may further include a thermal sensor. The thermal sensor may be configured of a pair of thermocouples embedded in the main body part of the catheter and is used for measuring the temperature of a living tissue in the vicinity of the light emission part. This enables real time observation of changes in temperature of a living tissue due to light irradiation, and thus can contribute to the suppression of blood coagulation or damages to living tissues due to light irradiation.

For example, the inner surface of the distal tip of the catheter and the distal end surface of the distal tip of the light irradiation device may be formed of a magnetic material, so as to attract each other. This enables to easily maintain a state in which the inner surface of the distal tip of the catheter and distal end surface of the distal tip of the light irradiation device are pressed to each other (the state in Fig. 2), when the light irradiation device is inserted into the catheter.

For example, at least one of the catheter and the light irradiation device may further include a radiopaque marker part. In the catheter, the marker part is preferably provided in the vicinity of the light emission part. Further, in the light irradiation device, the marker part is preferably provided in the vicinity of the light irradiation part. In this manner, an operator can easily position a light irradiation site (the light emission part and the light irradiation part) in a biological lumen through the confirmation of the position of the marker part in vivo by radiography. Further, in the configurations according to the fourth to the tenth embodiment, the marker part may be provided in the vicinity of the first to the sixth expansion/contraction parts.

### [Modification example 2]

In the above first to the twelfth embodiment, examples of the configurations of the first opening 161, the second opening 171, and the third opening 171B to be formed in the main body part are described. However, the configurations of the first to the third openings can be variously changed. For example, the first to the third openings may be configured of a plurality of openings that bring the inside and outside of the shaft into communication. For example, the first to the third openings may be configured of pores of a porous body embedded on the side of the shaft. For example, the releasing directions of a fluid from the first to the third openings may be not inclined to the side of the light emission part.

### [Modification example 3]

The above first to twelfth embodiment show examples of the configurations of the first to the sixth expansion/contraction parts 130 to 130I. However, the configurations of the first to the sixth expansion/contraction parts can be variously changed. For example, the first to the sixth expansion/contraction parts may be configured of mesh members expandable and contractable in the radial direction, which are mesh-shaped by weaving wires to create a mesh pattern. With such a mesh member, the light emission part can be fixed at a desired position in a biological lumen. Further, wires configuring the mesh member are formed of an optical fiber and the surface of the mesh member is processed to configure the light emission part, enabling light irradiation from a position closer to the inner wall of a biological lumen. This can suppress the absorption of irradiated light by a body fluid (e.g., blood) flowing within a biological lumen, enables to efficiently irradiate a target tissue with light and can contribute to the suppression of damages to a biological tissue due to unnecessary irradiation of a living tissue with light.

### [Modification example 4]

The above first to twelfth embodiments show examples of the configurations of the light emission parts 139 and 139B, and the light irradiation part 239. However, the configurations of the light emission part and the light irradiation part can be variously changed. For example, the configuration of the light emission part is not limited to the one by which the light inside the main body part is allowed to pass therethrough to the outside, and may be the one by which light irradiation (light is emitted) is performed to the outside from the outer surface of the main body part. In this case, the light emission part can be formed by subjecting the distal end part of an optical fiber joined to the outer surface of the main body part to known processes, in a manner similar to that described for the light irradiation part according to the first embodiment.

The above first and second embodiments illustrate a case in which a light irradiation part for irradiating a portion in the circumferential direction with light in combination with a light emission part provided on the entire perimeter. Further, the third embodiment illustrates a case in which a light irradiation part for irradiating a portion in the circumferential direction with light in combination with a light emission part provided at a portion in the circumferential direction. However, any combination of the light transmission range of the light emission part and the light irradiation range of the light irradiation part is possible. For example, a light emission part for allowing light to pass through the entire perimeter and a light irradiation part for performing light irradiation in all-around direction may be combined, a light emission part for allowing light to pass through the entire perimeter and a light irradiation part for irradiating a portion in the circumferential direction with light may be combined, a light emission part for allowing light to pass through a portion in the circumferential direction and a light irradiation part for performing light irradiation in all-around direction may be combined, and a light emission part for allowing light to pass through a portion in the circumferential direction and a light irradiation part for irradiating a portion in the circumferential direction with light may be combined.

### [Modification example 5]

The configurations of the catheters 1, and 1A to 1K and the light irradiation device 2 according to the above first to twelfth embodiments and the configurations of the catheters 1 and 1A to 1K and the light irradiation device 2 according to the above modification examples 1 to 4 may be combined as appropriate. For example, the catheter including the second opening according to the second embodiment may be provided with the third opening described in the third embodiment. For example, a catheter including all of the first to the third openings described in the first to the third embodiments may also be configured.

For example, the catheter described in the eleventh or the twelfth embodiments may be provided with the second opening described in the second embodiment or the third opening described in the third embodiment. For example, the catheter described in the eleventh or the twelfth embodiments may be provided with the first to the sixth expansion/contraction parts described in the fourth to the tenth embodiments.

In the above, the present aspects are described based on the embodiments and the modification examples. However, the embodiments of the aforementioned aspects are provided merely for clear understanding of the present aspects, and should not be construed as limiting to the present aspects. The invention is defined by the appended claims.

### DESCRIPTION OF REFERENCE NUMERALS

1, 1A to 1K...CATHETER
2...LIGHT IRRADIATION DEVICE
3....LASER LIGHT GENERATOR
30...LIGHT TRANSMISSION PART
39...LIGHT IRRADIATION PART
110, 110A to 110J...MAIN BODY PART
110L...INNER CAVITY
110o...OPENING
111, 111A, 111D to 111F, 111I...FIRST SHAFT
112, 112B to 112J...SECOND SHAFT
112o...OPENING
113, 113D to 113H... THIRD SHAFT
114, 114E...FOURTH SHAFT
120 ... DISTAL TIP
130...FIRST EXPANSION/CONTRACTION PART
130D...SECOND EXPANSION/CONTRACTION PART
130F...THIRD EXPANSION/CONTRACTION PART
130G...FOURTH EXPANSION/CONTRACTION PART
130H...FIFTH EXPANSION/CONTRACTION PART
130I...SIXTH EXPANSION/CONTRACTION PART
130K...EXPANSION/CONTRACTION PART
139, 139B..LIGHT EMISSION PART
140...CONNECTOR
140o...OPENING
142...BLADE
150, 150J...INNER SHAFT
150L, 150LJ...DEVICE LUMEN
151, 152... OPENING
160...FIRST INNER SHAFT
160L...FIRST FLUID LUMEN
161...FIRST OPENING
162...OPENING
170...SECOND INNER SHAFT
170B...THIRD INNER SHAFT
170L...SECOND FLUID LUMEN
170LB...THIRD FLUID LUMEN
171...SECOND OPENING
171B...THIRD OPENING
180, 180D, 180F to 180I, 180K...FOURTH INNER SHAFT
180L...EXPANSION MEDIUM LUMEN
181...FOURTH OPENING
191, 191B, 191E to 191I...FIRST SEALING PART
192, 192E to 192I...SECOND SEALING PART
193, 193D, 193E, 193G...THIRD SEALING PART
195, 195K...FIFTH SEALING PART
196K...SIXTH SEALING PART
197...SEVENTH SEALING PART
210...SECOND MAIN BODY PART
220 ... DISTAL TIP
239...LIGHT IRRADIATION PART
250...LIGHT TRANSMISSION PART

## Claims

1. A catheter (1, 1A-1K), comprising:
a main body part (110, 110A-110J) of long tubular shape;
a light emission part (139, 139B) that is provided on the side of the main body part (110, 110A-110J) and is configured to emit light in a predetermined outward direction; and
an opening (161, 171, 171B) that is provided on the side of the main body part (110, 110A-110J) and is capable of releasing a fluid,
wherein the opening includes a first opening (161) arranged on a proximal end side beyond the light emission part and/or a second opening (171) arranged on a distal end side beyond the light emission part (139, 139B), and
wherein the opening includes a third opening (171B) arranged at a position different in the circumferential direction from a position where light is configured to be emitted from the light emission part (139, 139B) on the side of the main body part,
wherein the fluid is releasable from the first opening (161) and/or the second opening (171) in such a manner that the direction (D1, D2) in which the fluid is releasable is inclined to the side of the light emission part (139, 139B),
the fluid is releasable from the third opening (171B) in such a manner that the direction (D3) in which the fluid is releasable is inclined to the side of the light emission part (139, 139B), and
the direction (D1-D3) in which the fluid is releasable from the first opening (161) and/or the second opening(171) and from the third opening (171B) intersects the direction (D0) in which light is configured to be emitted from the light emission part (139, 139B), wherein the direction (D3) in which the fluid is releasable from the third opening (171B) intersects the direction (DO) in which light is configured to be emitted from the light emission part (139, 139B) in the circumferential direction.

2. The catheter (1, 1A-1K) according to claim 1, wherein the opening includes the first opening (161) arranged on a proximal end side beyond the light emission part (139,139B), and the fluid is released releasable from the first opening (161) in such a manner that the direction in which the fluid is releasable is inclined to the side of the light emission part (139,139B).

3. The catheter (1A) according to claim 1, wherein the opening includes the second opening (171) arranged on a distal end side beyond the light emission part (139), and the fluid is releasable from the second opening (171) in such a manner that the direction in which the fluid is releasable is inclined to the side of the light emission part (139).

4. The catheter (1C,1E) according to claim 2, further comprising:
a first expansion/contraction part (130) that is provided in the main body part (110C,110E), arranged on the proximal end side beyond the first opening (161), and expandable and contractable in the radial direction.

5. The catheter (1D) according to claim 2 or 4, comprising:
a second expansion/contraction part (130D) that is provided in the main body part (110D), arranged on the distal end side beyond the light emission part (139), and expandable and contractable in the radial direction.

6. The catheter (1F) according to claim 3, further comprising:
a third expansion/contraction part (130F) that is provided in the main body part (110F), arranged on the distal end side beyond the second opening (171), and expandable and contractable in the radial direction.

7. The catheter (1G) according to claim 3 or 6, further comprising:
a fourth expansion/contraction part (130G) that is provided in the main body part (110G), arranged on the proximal end side beyond the light emission part (139), and expandable/contractable in the radial direction.

8. The catheter (1H) according to claim 1, further comprising:
a fifth expansion/contraction part (130H) that is provided in the main body part (110H), arranged on the proximal end side beyond the third opening (171B), and expandable and contractable in the radial direction.

9. The catheter (1I) according to claim 1 or 8, further comprising:
a sixth expansion/contraction part (130I) that is provided in the main body part (110I), arranged on the distal end side beyond the light emission part (139B), and expandable/contractable in the radial direction.

10. A light irradiation system, comprising:
the catheter (1,1A-1K) according to any one of claims 1 to 9; and a light irradiation device (2) that is insertable into the catheter (1,1A-1K), wherein
the light irradiation device (2) comprises a light irradiation part for light irradiation, which is provided at a position in proximity to the light emission part (139, 139B) when the light irradiation device (2) is inserted into the catheter (1, 1A-1K),
the light emission part (139, 139B) of the catheter (1, 1A-1K) is configured to light to the outside by allowing light irradiated by the light irradiation part to pass therethrough, and
the direction in which the fluid is releasable from the opening (161, 171, 171B) of the catheter (1, 1A-1K) intersects the direction in which light is configured to be emitted from the light emission part(139, 139B).

## Patentansprüche

1. Katheter (1, 1A-1K), umfassend:
einen Hauptkörperteil (110, 110A-110J) mit langer röhrenförmiger Gestalt;
einem Lichtemissionsabschnitt (139, 139B), der an der Seite des Hauptkörperabschnitts (110, 110A-110J) vorgesehen ist und so konfiguriert ist, dass er Licht in einer vorbestimmten Auswärtsrichtung emittiert; und
einer Öffnung (161, 171, 171B), die an der Seite des Hauptkörperabschnitts (110, 110A-110J) vorgesehen ist und zur Abgabe eines Fluids geeignet ist,
wobei die Öffnung eine erste Öffnung (161), die an einer proximalen Endseite jenseits des Lichtemissionsabschnitts angeordnet ist, und/oder eine zweite Öffnung (171) umfasst, die an einer distalen Endseite jenseits des Lichtemissionsabschnitts (139, 139B) angeordnet ist, und
wobei die Öffnung eine dritte Öffnung (171B) umfasst, die an einer Position angeordnet ist, die sich in Umfangsrichtung von einer Position unterscheidet, an der Licht aus dem Lichtemissionsabschnitt (139, 139B) an der Seite des Hauptkörperabschnitts emittiert werden soll,
wobei
das Fluid aus der ersten Öffnung (161) und/oder der zweiten Öffnung (171) derart austreten kann, dass die Richtung (D1, D2), in der das Fluid austreten kann, zur Seite des Lichtemissionsabschnitts (139, 139B) geneigt ist,
das Fluid aus der dritten Öffnung (171B) in einer solchen Weise freigegeben werden kann, dass die Richtung (D3), in der das Fluid freigegeben werden kann, zur Seite des Lichtemissionsabschnitts geneigt ist (139, 139B) geneigt ist, und
die Richtung (D1-D3), in der das Fluid aus der ersten Öffnung (161) und/oder der zweiten Öffnung (171) und aus der dritten Öffnung (171B) freisetzbar ist, die Richtung (D0) schneidet, in der Licht aus dem Lichtemissionsabschnitt (139, 139B) emittiert werden soll, wobei die Richtung (D3), in der das Fluid aus der dritten Öffnung (171B) austreten kann, die Richtung (DO) schneidet, in der Licht aus dem Lichtemissionsabschnitt (139, 139B) in Umfangsrichtung emittiert werden soll.

2. Katheter (1, 1A-1K) gemäß Anspruch 1, wobei die Öffnung die erste Öffnung (161) umfasst, die an einer proximalen Endseite jenseits des Lichtemissionsabschnitts (139, 139B) angeordnet ist, und das Fluid aus der ersten Öffnung (161) derart freisetzbar ist, dass die Richtung, in der das Fluid freisetzbar ist, zur Seite des Lichtemissionsabschnitts (139, 139B) geneigt ist.

3. Katheter (1A) gemäß Anspruch 1, wobei die Öffnung die zweite Öffnung (171) umfasst, die auf einer distalen Endseite jenseits des Lichtemissionsabschnitts (139) angeordnet ist, und das Fluid aus der zweiten Öffnung (171) in einer solchen Weise freisetzbar ist, dass die Richtung, in der das Fluid freisetzbar ist, zur Seite des Lichtemissionsabschnitts (139) geneigt ist.

4. Katheter (1C, 1E) nach Anspruch 2, der ferner umfasst:
einen ersten Expansions-/Kontraktionsabschnitt (130), der in dem Hauptkörperabschnitt (110C, 110E) vorgesehen ist, auf der proximalen Endseite jenseits der ersten Öffnung (161) angeordnet ist und in radialer Richtung expandierbar und kontrahierbar ist.

5. Katheter (1D) nach Anspruch 2 oder 4, umfassend:
einen zweiten Expansions-/Kontraktionsabschnitt (130D), der im Hauptkörperabschnitt (110D) vorgesehen ist, auf der distalen Endseite jenseits des Lichtemissionsabschnitts (139) angeordnet ist und in radialer Richtung expandierbar und kontrahierbar ist.

6. Katheter (1F) nach Anspruch 3, der ferner umfasst:
einen dritten Expansions-/Kontraktionsabschnitt (130F), der im Hauptkörperabschnitt (110F) vorgesehen ist, auf der distalen Endseite jenseits der zweiten Öffnung (171) angeordnet ist und in radialer Richtung expandierbar und kontraktierbar ist.

7. Katheter (1G) nach Anspruch 3 oder 6, der ferner umfasst:
einen vierten Expansions-/Kontraktionsabschnitt (130G), der im Hauptkörperabschnitt (110G) vorgesehen ist, auf der proximalen Endseite jenseits des Lichtemissionsabschnitts (139) angeordnet ist und in radialer Richtung expandierbar/kontrahierbar ist.

8. Der Katheter (1H) gemäß Anspruch 1, der ferner umfasst:
ein fünftes Expansions-/Kontraktionselement (130H), das im Hauptkörperelement (110H) vorgesehen ist, auf der proximalen Endseite jenseits der dritten Öffnung (171B) angeordnet ist und in radialer Richtung expandierbar und kontraktierbar ist.

9. Katheter (11) nach Anspruch 1 oder 8, der ferner umfasst:
einen sechsten Expansions-/Kontraktionsabschnitt (130I), der im Hauptkörperabschnitt (110I) vorgesehen ist, auf der distalen Endseite jenseits des Lichtemissionsabschnitts (139B) angeordnet ist und in radialer Richtung expandierbar/kontrahierbar ist.

10. Ein Lichtbestrahlungssystem, umfassend:
den Katheter (1, 1A-1K) gemäß einem der Ansprüche 1 bis 9; und
eine Lichtbestrahlungsvorrichtung (2), die in den Katheter (1, 1A-1K) einführbar ist, wobei
die Lichtbestrahlungsvorrichtung (2) einen Lichtbestrahlungsteil zur Lichtbestrahlung umfasst, der an einer Position in der Nähe des Lichtemissionsteils (139, 139B) vorgesehen ist, wenn die Lichtbestrahlungsvorrichtung (2) in den Katheter (1, 1A-1K) eingeführt ist,
der Lichtemissionsabschnitt (139, 139B) des Katheters (1, 1A-1K) so konfiguriert ist, dass er Licht nach außen emittiert, indem er das vom Lichtbestrahlungsabschnitt bestrahlte Licht durchlässt, und die Richtung, in der die Flüssigkeit aus der Öffnung (161, 171, 171B) des Katheters (1, 1A-1K) austreten kann, schneidet die Richtung, in die Licht von dem Lichtemissionsabschnitt (139, 139B) emittiert werden soll.

## Revendications

1. Cathéter (1, 1A-1K), comprenant :
une partie de corps principal (110, 110A-110J) de forme tubulaire allongée ;
une partie d'émission de lumière (139, 139B) qui est prévue sur le côté de la partie de corps principal (110, 110A-110J) et qui est configurée pour émettre de la lumière dans une direction vers l'extérieur prédéterminée ; et
une ouverture (161, 171, 171B) qui est prévue sur le côté de la partie de corps principal (110, 110A-110J) et capable de libérer un fluide,
dans lequel l'ouverture comprend une première ouverture (161) disposée sur un côté d'extrémité proximale au-delà de la partie d'émission de lumière et/ou une deuxième ouverture (171) disposée sur un côté d'extrémité distale au-delà de la partie d'émission de lumière (139, 139B), et
dans lequel l'ouverture comprend une troisième ouverture (171B) disposée à une position différente dans la direction circonférentielle d'une position où la lumière est configurée pour être émise à partir de la partie d'émission de lumière (139, 139B) sur le côté de la partie principale du corps,
dans lequel
le fluide peut être libéré à partir de la première ouverture (161) et/ou de la deuxième ouverture (171) de telle manière que la direction (D1, D2) dans laquelle le fluide peut être libéré est inclinée vers le côté de la partie d'émission de lumière (139, 139B),
le fluide peut être libéré à partir de la troisième ouverture (171B) de telle manière que la direction (D3) dans laquelle le fluide peut être libéré soit inclinée vers le côté de la partie d'émission de lumière (139, 139B), et
la direction (D1-D3) dans laquelle le fluide peut être libéré à partir de la première ouverture (161) et/ou de la deuxième ouverture (171) et à partir de la troisième ouverture (171B) croise la direction (D0) dans laquelle la lumière est configurée pour être émise à partir de la partie d'émission de lumière (139, 139B), dans lequel la direction (D3) dans laquelle le fluide peut être libéré depuis la troisième ouverture (171B) croise la direction (DO) dans laquelle la lumière est configurée pour être émise depuis la partie d'émission de lumière (139, 139B) dans la direction circonférentielle.

2. Le cathéter (1, 1A-1K) selon la revendication 1, dans lequel l'ouverture comprend la première ouverture (161) disposée sur un côté d'extrémité proximale au-delà de la partie d'émission de lumière (139, 139B), et le fluide peut être libéré à partir de la première ouverture (161) de telle manière que la direction dans laquelle le fluide peut être libéré est inclinée vers le côté de la partie d'émission de lumière (139, 139B).

3. Le cathéter (1A) selon la revendication 1, dans lequel l'ouverture comprend la deuxième ouverture (171) disposée sur un côté d'extrémité distale au-delà de la partie d'émission de lumière (139), et le fluide peut être libéré de la deuxième ouverture (171) de telle manière que la direction dans laquelle le fluide peut être libéré est inclinée vers le côté de la partie d'émission de lumière (139).

4. Le cathéter (1C, 1E) selon la revendication 2, comprenant en outre :
une première partie d'expansion/contraction (130) qui est prévue dans la partie de corps principal (110C, 110E), disposée sur le côté d'extrémité proximale au-delà de la première ouverture (161), et pouvant être expansée et contractée dans la direction radiale.

5. Le cathéter (1D) selon la revendication 2 ou 4, comprenant :
une deuxième partie d'expansion/contraction (130D) qui est prévue dans la partie de corps principal (110D), disposée sur le côté d'extrémité distale au-delà de la partie d'émission de lumière (139), et pouvant être expansée et contractée dans la direction radiale.

6. Le cathéter (1F) selon la revendication 3, comprenant en outre :
une troisième partie d'expansion/contraction (130F) qui est prévue dans la partie de corps principal (110F), disposée sur le côté d'extrémité distale au-delà de la deuxième ouverture (171), et pouvant être dilatée et contractée dans la direction radiale.

7. Le cathéter (1G) selon la revendication 3 ou 6, comprenant en outre :
une quatrième partie d'expansion/contraction (130G) qui est prévue dans la partie de corps principal (110G), disposée sur le côté d'extrémité proximale au-delà de la partie d'émission de lumière (139), et pouvant être expansée/contractée dans la direction radiale.

8. Le cathéter (1H) selon la revendication 1, comprenant en outre :
une cinquième partie d'expansion/contraction (130H) qui est prévue dans la partie de corps principal (110H), disposée sur le côté d'extrémité proximale au-delà de la troisième ouverture (171B), et pouvant être dilatée et contractée dans la direction radiale.

9. Le cathéter (11) selon la revendication 1 ou 8, comprenant en outre :
une sixième partie d'expansion/contraction (1301) qui est prévue dans la partie de corps principal (1101), disposée sur le côté d'extrémité distale au-delà de la partie d'émission de lumière (139B), et pouvant être expansée/contractée dans la direction radiale.

10. Système d'irradiation lumineuse, comprenant :
le cathéter (1, 1A-1K) selon l'une quelconque des revendications 1 à 9 ; et
un dispositif d'irradiation lumineuse (2) pouvant être inséré dans le cathéter (1, 1A-1K), dans lequel
le dispositif d'irradiation lumineuse (2) comprend une partie d'irradiation lumineuse pour l'irradiation lumineuse, qui est prévue à une position à proximité de la partie d'émission lumineuse (139, 139B) lorsque le dispositif d'irradiation lumineuse (2) est inséré dans le cathéter (1, 1A-1K),
la partie d'émission de lumière (139, 139B) du cathéter (1, 1A-1K) est configurée pour émettre de la lumière vers l'extérieur en laissant passer la lumière irradiée par la partie d'irradiation lumineuse, et la direction dans laquelle le fluide peut être libéré depuis l'ouverture (161, 171, 171B) du cathéter (1, 1A-1K) croise la direction dans laquelle la lumière est configurée pour être émise à partir de la partie d'émission de lumière (139, 139B).
